# EUROPÄISCHE PATENTSCHRIFT

(11) **EP 2 976 159 B1**
(45) Veröffentlichungstag und Bekanntmachung des Hinweises auf die Patenterteilung: **02.05.2018**
(21) Anmeldenummer: 14714624.5
(22) Anmeldetag: 21.03.2014
(51) Int. Cl.: B05B 13/00, C12M 1/00, C12M 1/34

(54) **VORRICHTUNG UND VERFAHREN ZUR BENETZUNG EINER PROBE MIT EINEM AEROSOL**
DEVICE AND METHOD FOR WETTING A SAMPLE WITH AN AEROSOL
APPAREIL ET MÉTHODE POUR MOUILLER UN ÉCHANTILLON AVEC UN AÉROSOL

(30) Priorität: 22.03.2013 DE 102013005010
(43) Veröffentlichungstag der Anmeldung: 27.01.2016
(73) Patentinhaber: Helmholtz Zentrum München - Deutsches Forschungszentrum für Gesundheit und Umwelt (GmbH), 85764 Neuherberg (DE)
(72) Erfinder: SCHMID, Otmar, 80639 München (DE); LENZ, Anke-G., 80805 München (DE); LENTNER, Bernd, 85435 Erding (DE); EICKELBERG, Oliver, 80798 München (DE)
(74) Vertreter: Schiweck, Weinzierl & Koch Patentanwälte Partnerschaft mbB
(86) Internationale Anmeldenummer: PCT/EP2014/055722
(87) Internationale Veröffentlichungsnummer: WO 2014/147229

(56) Entgegenhaltungen:
- DE-A1-102009 016 364
- JP-A- H08 112 560
- PAUR ET AL.: "In-vitro cell exposure studies for the assessment of nanoparticle toxicity in the lung - A dialog between aerosol science and biology", JOURNAL OF AEROSOL SCIENCE, Bd. 42, 2011, Seiten 668-692, XP0028252425, in der Anmeldung erwähnt
- LENZ, A.G.; E. KARG; B. LENTNER; V. DITTRICH; C. BRANDENBERGER; B. ROTHEN-RUTISHAUSER; H. SCHULZ; G. A. FERRON; O. SCHMID: "A dose-controlled system for air-liquid interface cell exposure and application to zinc oxide nanoparticles", PARTICLE AND FIBRE TOXICOLOGY, Bd. 6, Nr. 32, 2009, Seiten 1-17, XP002725077, in der Anmeldung erwähnt
- BLANK F; ROTHEN-RUTISHAUSER BM; SCHURCH S; GEHR P: "An optimized in vitro model of the respiratory tract wall to study particle cell interactions", JOURNAL OF AEROSOL MEDICINE-DEPOSITION CLEARANCE AND EFFECTS IN THE LUNG, Bd. 19, Nr. 3, 2006, Seiten 392-405, XP002725078, in der Anmeldung erwähnt
- BRANDENBERGER C ET AL: "Effects and uptake of gold nanoparticles deposited at the air-liquid interface of a human epithelial airway model", TOXICOLOGY AND APPLIED PHARMACOLOGY, ACADEMIC PRESS, AMSTERDAM, NL, vol. 242, no. 1, 1 January 2010 (2010-01-01), pages 56-65, XP026765572, ISSN: 0041-008X, DOI: 10.1016/J.TAAP.2009.09.014 [retrieved on 2009-09-29]

## Beschreibung

### Gebiet der Erfindung

Die vorliegende Erfindung betrifft eine Vorrichtung zur Benetzung einer Probe mit einem Aerosol, bevorzugt eine biologische Probe, beispielsweise eine Zellprobe, insbesondere an der Luft-Flüssigkeitsgrenzschicht. Weiter betrifft die Erfindung auch ein entsprechendes Verfahren zur Benetzung einer Probe mit einem Aerosol. Bei der Probe handelt es sich insbesondere um Zellen und vorzugsweise um Epithelzellen, besonders bevorzugt um Lungenzellen.

### Stand der Technik

Oftmals ist es notwendig, eine Probe, beispielsweise Zellen, in Experimenten einer Substanz und insbesondere einer pharmazeutisch wirksamen Substanz, bspw. für das Wirkstoffscreening, oder einer toxischen Substanz, bspw. für Toxizitätsuntersuchungen, auszusetzen.

Üblicherweise werden dazu in vitro Zellversuche durchgeführt. Hierbei wird ein Wirkstoff bzw. ein toxikologisch wirksamer Stoff einer Probe von Zellen unter sogenannten submersen Zellkulturbedingungen zugesetzt. Bei solchen submersen Zellkulturbedingungen sind die Zellen vollständig vom Zellmedium bedeckt und die Testsubstanz, beispielsweise eine pharmazeutisch wirksame oder eine toxische Substanz, wird direkt in das Zellmedium einpipettiert.

Für Epithelzellen, also Zellen, die den Körper nach außen begrenzen, beispielsweise Lungenepithelzellen, Hautzellen oder Retinazellen, ist diese Situation allerdings unrealistisch, da die Epithelzellen im Körper typischerweise eine Luft-Flüssigkeits-Grenzschicht (LFG) bilden. Das heißt auf der einen Seite der Epithelzellen befindet sich Luft bzw. allgemeiner Gas und auf der anderen Seite interstitielles Gewebe bzw. allgemeiner Flüssigkeit.

Für Studien und Experimente an solchen Zellen ist das vollständige Eintauchen solcher Zellen in das Zellmedium physiologisch unrealistisch. Gleichzeitig sind Studien bzw. Experimente an solchen Zellen aber oftmals von Interesse. Dies gilt insbesondere für Wirksamkeits- und Toxizitätsstudien mit inhalativ verabreichten Stoffen, beispielsweise für Inhalationstherapien bzw. Studien zur Arbeitsplatzsicherheit im Umgang mit aerosolisierbaren Materialien insbesondere Nanomaterialien.

Untersuchungen an der LFG werden beispielsweise eingesetzt, um die Einwirkungen von aerosolgetragenen Substanzen (z.B. Wirkstoffe, Toxine, Nanopartikel) auf Zellen zu analysieren. Hierbei steht insbesondere die Einwirkung auf Zellen im Vordergrund, die sich an der Kontaktfläche Luft/Körperflüssigkeiten befinden, wie beispielsweise Epithelzellen der Lunge, Haut oder der Retina. Bei der Inhalationstherapie werden aerosolisierte Wirkstoffe gezielt auf das Lungenepithel aufgebracht, um Lungenerkrankungen oder systemische Erkrankungen zu behandeln. Darüber hinaus bietet die Lunge auch das wichtigste Einfallstor für den Umgang mit vielen toxischen oder potentiell toxischen Stoffen wie z.B. Umweltfeinstaub oder Nanomaterialien am Arbeitsplatz.

Um solche epithelialen Zellen unter möglichst physiologischen Bedingungen untersuchen zu können, wurden verschiedene Methoden entwickelt, die ein solches LFG-Milieu simulieren. Insbesondere mittels Transwell-Inserts können Zellen an der LFG auch in standardisierten Mikrotiterplatten kultiviert werden. Das Kultivieren von Epithelzellen an der LFG ist grundsätzlich physiologisch realistischer und daher hinsichtlich der Wirkung von Substanzen möglicherweise aussagekräftiger als vorstehend beschriebene submerse Zellkulturen.

Es besteht daher ein grundsätzlicher Bedarf, ein geeignetes System für Studien und Experimente an derartigen LFG-Zellkulturen bereitzustellen, mit dem man einen Stoff gleichmäßig in geringer Schichtdicke (bspw. ca. 10-100 µm) auf LFG-Zellkulturen auftragen kann. Solche Stoffe umfassen typischerweise flüssige aerosolisierte Wirkstoffe, können aber auch trockene Substanzen umfassen. Insbesondere für pharmazeutische Studien mit z.T. sehr teuren Wirkstoffen sind eine hohe Depositionseffizienz und eine gleichmäßige Verteilung des aerosolisierten Wirkstoffs auf die LFG-Zellen von Vorteil. Da weder eine geeignete Vorrichtung noch ein entsprechendes Verfahren bisher etabliert sind, ist der Einsatz der LFG-Zellkulturen für pharmazeutische Studien und Experimente derzeit noch relativ eingeschränkt.

Es gibt bereits einige Systeme, die darauf abzielen, (Lungen)Zellen an der LFG mit aerosolisierten Stoffen zu belegen. Diese können, ausgehend vom verwendeten Lösungsansatz für die folgenden technologischen Herausforderungen, in verschiedene Kategorien eingeteilt werden. Einerseits stellt sich die Herausforderung des Transports der aerosolisierten Substanz zu den Zellen (Transport) und andererseits die Aufgabe der Deposition des Aerosols auf die Zellen (Deposition).

Gemäß dem System der DE102009016364A1 wird Aerosol durch einen Luftstrom zu den Zellen befördert (Transport: Luftströmung) und mittels Diffusion und/oder Sedimentation (je nach Größe und Masse des Aerosols) auf die Zellen deponiert.

Weiterhin ist es bekannt, Luftströmung zum Transport eines Aerosols zu verwenden, wobei die Deposition durch elektrostatische Abscheidung erfolgt. Hier werden die Aerosole zunächst elektrostatisch aufgeladen und dann in einem elektrischen Feld elektrophoretisch auf die Zellen deponiert.

Auch ist es bekannt, die Zellen aus relativ geringer Entfernung durch direktes "Ansprayen" aufzubringen. Hier erfolgt der Transport durch die Erzeugung von Aerosolen mit einer hohen Geschwindigkeit (z.B. mittels einer Hochdruckdüse oder eines schnellen Trägerluftstroms) und die Deposition auf den Zellen durch inertiale Impaktion. Diese Art der Systeme hat häufig den Nachteil, dass die gleichmäßige Verteilung der Aerosole auf den Zellen nicht gewährleistet ist oder dass der Betrieb technisch sehr aufwendig ist.

Oftmals sind die bekannten Systeme nur für toxikologisch relevante Trockenaerosole, nicht jedoch für pharmazeutische Flüssigkeitsaerosole verwendbar. Außerdem sind die verwendbaren Partikelgrößen oftmals eingeschränkt, bspw. auf unter 1 µm. Größere Aerosole von bspw. größer als 1 µm, wie sie für die Inhalationstherapie verwendet werden, sind oftmals nicht möglich.

Auch haben viele der bekannten Systeme eine sehr geringe Wirkstoffdepositionsrate, von bspw. etwa 0,1 µg/cm²/h bzw. etwa 0,1 nl/cm²/h für wässrige Lösungen (Paur et al. Journal of Aerosol Science 42 (2011) 668-692, und hierein Table 3), und benötigen dadurch sehr lange Expositionszeiten, von mehreren Stunden bis hin zu einigen Tagen, um messbare zellbiologische Antworten hervorzurufen. Entsprechende Verfahren und Apparaturen sind sehr kosten- und zeitintensiv und erschweren zudem die Arbeit mit Zellen, die nur für etwa 7 Tage kultiviert werden können. Auch der Betrieb sowie die Qualitätskontrolle gestalten sich aufwändig. Die (Zell)-Depositionseffizienz (prozentuale Anteil des investierten Materials im Aerosolgenerator, der auf die Zellen deponiert wird) der bekannten Systeme liegt in der Regel weit unter 100%, bspw. bei unter 1%, was mitunter auf eine geringe Abscheideeffizienz der Aerosole auf den Zellen und auf Substanzverluste (-rückstände) im Aerosolgenerator und in den Zuführungsleitungen zurückzuführen ist. Elektrostatische Depositionssysteme erweisen sich zudem als nachteilig, da die nicht zu vermeidenden Ladungsströme einen verfälschenden Einfluss auf die Zellantwort haben könnten. Zudem sind einige der bekannten Systeme dahingehend nachteilig, dass sie keine gleichmäßige Verteilung oder Gleichverteilung des Stoffes auf den Zellen erlauben. Somit ist insbesondere keine vergleichbare und reproduzierbare Dosimetrie auf den Zellen möglich. Aufgrund der unkontrollierten Bedingungen ist eine verlässliche Dosis-Wirkungsbeziehung nur schwierig zu bestimmen. Schließlich sind Lösungen, die den Stoff durch inertiale Impaktion deponieren, nachteilig, da die Zellen unter anderem unter den hohen Anströmgeschwindigkeiten des Aerosols leiden.

Den meisten bekannten Systemen ist gemein, dass das Aerosol mittels eines kontinuierlichen Luftstroms in eine Expositionskammer getragen wird, was einen entsprechenden technologischen Aufwand bedingt. Beispielhaft für ein solches System ist das Air-Liquid-Interface Cell Exposure System, kurz ALICE (beschrieben in Lenz, A.G., E. Karg, B. Lentner, V. Dittrich, C. Brandenberger, B. Rothen-Rutishauser, H. Schulz, G. A. Ferron und O. Schmid, A dose-controlled system for air-liquid interface cell exposure and application to zinc oxide nanoparticles, Particle and Fibre Toxicology 6 (32), 1-17, 2009). Eine Aerosolwolke wird mittels eines externen Luftstroms von der einen Seite in eine Expositionskammer hineintransportiert, dort fällt die Aerosolwolke nach unten, formt einen Vortex und bildet einen Nebel, der auf die Zellen absedimentiert und diese dadurch mit Substanz benetzt. Die aerosol-entleerte Luft wird auf der anderen Seite der Expositionskammer wieder abgezogen. Dieses System weist eine relativ geringe Zelldepositionseffizient von ca. 7% auf und nimmt relativ viel Platz (ca. 1 m³) in Anspruch und kann deswegen nicht unter einer Sterilbank betrieben werden. Weiterhin ist es technisch sehr kompliziert aufgebaut und deswegen aufwendiger im Betrieb. U.a benötigt das System einen Luftbefeuchter, eine Pumpe und einen Luftflussmeter zur Erzeugung eines externen Luftflusses sowie eine Tröpfchenfalle, um Störungen im Expositionsablauf zu vermeiden. Darüber hinaus ist der Vernebler seitlich neben der Expositionskammer angeordnet und das System wird mit einem externen Luftfluss, insbesondere also nicht luftstrom-frei, betrieben.

Ein anderes Verfahren, das lufstrom-frei betrieben wird, wurde von F. Blank vorgestellt in Blank F, Rothen-Rutishauser BM, Schurch S, Gehr P: An optimized in vitro model of the respiratory tract wall to study particle cell interactions. Journal of Aerosol Medicine-Deposition Clearance and Effects in the Lung, 19(3):392-405, 2006. In diesem Verfahren wird eine Probe durch direktes Ansprayen benetzt, wobei der Sprayer 12 cm oberhalb der Probe positioniert ist. Das System erzeugt ein Aerosolspray und deponiert das Aerosol mittels inertialer Impaktion auf der Probe. Es wird keine Sedimentation und es werden keine Wolkeneffekte genutzt oder nutzbar gemacht. Außerdem ist das System offen (ohne Seitenwände oder Deckelbereich) und es wird durch das hier bereitgestellte Verfahren nur eine nicht benannte aber wahrscheinlich relativ geringe Depositionseffizienz erreicht. Weiterhin weist ein solches Spray eine Tröpfchenverteilung auf, die schon nach einer sehr geringern Distanz vom Ort der Erzeugung heterogen ist. Daher ist ein solches Verfahren nicht geeignet, um eine homogene Verteilung von Substanzen auf Proben, die über eine Fläche von 100cm² oder mehr angeordnet sind, bereitzustellen. Darüber hinaus ist die Reproduzierbarkeit der Benetzung der Proben nicht gewährleistet, da der verwendete Vernebler (Microsprayer, Penn-Century Inc., USA) manuell betrieben wird.

Es ist daher eine erste Aufgabe der vorliegenden Erfindung, eine alternative und vorzugsweise verbesserte Vorrichtung bzw. ein verbessertes Verfahren bereitzustellen. Diese Vorrichtung bzw. dieses Verfahren sollen insbesondere die oben genannten Probleme abschwächen oder lösen.

Mit anderen Worten ist es daher eine Aufgabe der vorliegenden Erfindung, eine Vorrichtung bzw. ein Verfahren zur raschen, einfachen, gleichmäßigen und/oder reproduzierbaren Aufbringung einer Substanz bzw. eines Aerosols (insbesondere einer flüssigen oder festen Substanz) auf eine Probe, insbesondere auf ein LFG-Zellkultur, bereitzustellen. Ferner sollte diese Vorrichtung bzw. dieses Verfahren dazu geeignet sein, dass das Aerosol mit einer hohen Depositionsrate (Aerosolmasse pro Zeit pro zellbedeckter Fläche) aufgetragen wird.

Ebenso ist es eine Aufgabe der vorliegenden Erfindung, diese Vorrichtung bzw. dieses Verfahren effizient, einfach handhabbar und dosimetrisch genau auszugestalten. Bevorzugt sollte das Verfahren selbst keine biologischen Effekte bei den Zellen oder an Gewebeschnitten hervorrufen.

### Zusammenfassung der Erfindung

Diese Aufgabe wird bzw. diese Aufgaben werden von der erfindungsgemäßen Vorrichtung und den erfindungsgemäßen Verfahren gelöst, insbesondere wie in den beigefügten Ansprüchen, den nachfolgenden Aspekten und/oder der weiteren Beschreibung beschrieben. Insbesondere betrifft die vorliegende Erfindung ein neuartiges System (Vorrichtung, Verfahren und Verwendung) zur Aerosolexposition von einer Probe. Bei dieser Probe kann es sich um eine biologische Probe handeln, beispielsweise um Zellen. Diese Zellen können in Form von Einzelzellen, als Zellenverband (bis hin zu Organen), Gewebeschnitte und/oder Kulturzellen, beispielsweise als Rasen von Kulturzellen, vorliegen. Insbesondere kann es sich um Zellen an der Luft-Flüssigkeits-Grenzschicht (LFG) handeln. Weiterhin kann es sich bei dieser Probe aber auch um eine Werkstoffprobe, beispielsweise aus Metall, Kunststoff und/oder Glas handeln.

Gemäß einem ersten Aspekt umfasst die Erfindung eine Vorrichtung zur Benetzung mindestens einer Probe mittels eines Aerosols. Unter einem Aerosol wird hierbei ein Gemisch aus einer Substanz mit einem Gas, beispielsweise Luft, verstanden. Die Substanz kann in flüssiger oder fester Form vorliegen, beispielsweise als Tröpfchen oder Staub. In anderen Worten handelt es sich bei einem Aerosol also um ein Gemisch aus festen und/oder flüssigen Schwebeteilchen und einem Gas. Die Vorrichtung umfasst Seitenwände, die vorzugsweise im Wesentlichen vertikal sind, sowie einen Bodenbereich. Der Bodenbereich ist im normalen Gebrauch, insbesondere im Hinblick auf die Gravitationsrichtung, im unteren Bereich der Vorrichtung angeordnet. Er wird vorzugsweise durch Seitenwände, insbesondere seitlich, begrenzt. Der Bodenbereich ist vorzugsweise zur Aufnahme oder zum Zusammenwirken mit einer Positionierungsvorrichtung zur Positionierung der Probe angepasst oder weist eine solche auf. Die Vorrichtung umfasst ferner einen Deckelbereich, der dem Bodenbereich vertikal gegenüberliegt (oben), und der vorzugsweise durch die vertikalen Seitenwänden, insbesondere seitlich, begrenzt ist. Der Deckelbereich ist vorzugsweise zur Aufnahme oder zum Zusammenwirken mit einem Deckel bzw. einer Decke angepasst oder weist einen solchen auf. Die Vorrichtung bildet eine Aerosolexpositionskammer. Unter dem Begriff Expositionskammer wird insbesondere der Raum verstanden, der durch die Vorrichtung definiert, und der insbesondere durch die Seitenwände, den Bodenbereich und den Deckelbereich von der Umgebung getrennt ist. Sie umfasst ferner und/oder ist ausgebildet zum Zusammenwirken mit einer Vorrichtung zur Aerosolgeneration, die auch als Aerosolisierungsvorrichtung bezeichnet wird, vorzugsweise einer Vernebelungsvorrichtung zur Erzeugung eines flüssigen Aerosols. Eine Vorrichtung zur Aerosolgeneration ist eine Vorrichtung, die je nach Ausformung und Verwendung einen Nebel, eine Wolke oder ein Tröpfchenaerosol erzeugen kann. Die Aerosolisierungsvorrichtung ist dabei vorzugsweise in der Nähe des Deckelbereichs angeordnet und insbesondere im, oberhalb oder unterhalb des Deckelbereichs bzw. Deckels, vorzugsweise jedenfalls in Draufsicht innerhalb bzw. zwischen der/den Seitenwände(n). Die Vorrichtung und/oder die Aerosolisierungsvorrichtung ist bzw. sind dabei derart angepasst, dass das Aerosol in Richtung des Bodenbereichs freisetzbar ist. Die Aerosolisierungsvorrichtung ist derart über dem Bodenbereich angeordnet bzw. kann dort derart angeordnet werden, dass die Aerosolisierungsvorrichtung den Bodenbereich in einer vertikalen Draufsicht zumindest teilweise überdeckt und vorzugsweise in etwa mittig zu diesem angeordnet ist. Die Vorrichtung weist vorzugsweise geeignete Mittel zur Positionierung und/oder Befestigung der Aerosolisierungsvorrichtung auf. Durch die erfindungsgemäße Vorrichtung kann die Probe mit einer Substanz benetzt werden.

Die Positionierungsvorrichtung erlaubt es insbesondere, mindestens eine Probe, insbesondere eine Zellprobe und bevorzugt Epithelzellen, bevorzugt an der LFG geeignet anzuordnen. Die Positionierungsvorrichtung ist dabei vorzugsweise plattenartig ausgebildet und weist vorzugsweise eine oder mehrere Vertiefungen für die Probe bzw. Probenhalterung auf. Ein bevorzugtes Beispiel für eine Positionierungsvorrichtung ist eine Mikrotiterplatte. Weitere Beispiele sind Petrischalen oder Objektträger. Die Vertiefungen der Positioniervorrichtung sind vorzugsweise ausgebildet, ein Nährmedium für Zellen sowie eine Positionierhilfe, insbesondere Transwell-Inserts, aufzunehmen, mit deren Hilfe die Zellen in der LFG angeordnet werden können.

Der Bodenbereich der Vorrichtung ist ausgebildet, eine Positionierungsvorrichtung aufzunehmen oder mit einer solchen zusammenzuwirken. Dazu ist der Bodenbereich beispielsweise ausgebildet, eine Positionierungsvorrichtung seitlich zu umschließen oder Platz für eine Positionierungsvorrichtung zu bieten. Der Bodenbereich kann eine Bodenplatte aufweisen, die die durch die Seitenwände gebildete Bodenöffnung ganz oder zumindest teilweise schließt. Die Positionierungsvorrichtung kann auf der Bodenplatte angeordnet werden. Die Positionierungsvorrichtung kann beispielsweise als eine, zwei, drei oder auch mehr als drei Mikrotiterplatten ausgestaltet sein. Der Bodenbereich ist dazu vorzugsweise zur Aufnahme einer solchen Anzahl an Mikrotiterplatten ausgebildet, vorzugsweise durch eine entsprechende Bodenplatte mit einer Vertiefung zur Platzierung der Mikrotiterplatte, die an die Geometrie der Seitenwände angepasst ist. Dies kann besonders vorteilhaft sein, da die vorgeschriebene Vorrichtung dann mit bestehenden Systemen verwendet werden kann. Vorzugsweise sind dies Mikrotiterplatten. Die Positioniervorrichtung kann jedoch auch individuell ausgestaltet sein. Vorzugsweise umfasst die Positionierungsvorrichtung Elemente zur Messung und/oder Regulierung einer Temperatur, vorzugsweise mit Hilfe eines Wasserkreislaufes oder einer elektrischen Temperiervorrichtung, beispielsweise einer Heizung, Elemente zur Versorgung der Probe, bspw. mit Nährmedium, und/oder Elemente zum Messen des Gewichts des deponierten Aerosols, vorzugsweise mit Hilfe einer oder mehreren Quartzkristall-Mikrowaage(n). Eine solche Quarzkristall-Mikrowaage eignet sich vorzugsweise und insbesondere zur direkten, in situ (real-time) Bestimmung der auf die Probe deponierten Substanzmenge. Die Positioniervorrichtung ist vorzugsweise zur Benetzung von 6, 12, 24, 48, 96, 384 oder 1536 Proben, bzw. 2 x (6, 12, 24, 48, 96, 384 oder 1536) oder 3 x (6, 12, 24, 48, 96, 384 oder 1536) oder 4 x (6, 12, 24, 48, 96, 384 oder 1536) Proben, ausgebildet.

Die hier beschriebene Vorrichtung ermöglicht es, ein Aerosol, geeignet und vorteilhaft auf einer Probe zu deponieren und diese zu benetzen. Insbesondere ermöglicht die Vorrichtung auf einfache und effiziente Weise eine rasche und homogene Benetzung einer und besonders mehrerer Proben. Dabei erweisen sich insbesondere der Aufbau der Vorrichtung bzw. der Expositionskammer sowie die Positionierung der Aerosolisierungsvorrichtung im Verhältnis zur Vorrichtung sowie zur Probe als vorteilhaft. Insbesondere erlaubt die Vorrichtung durch die Aerosolisierungsvorrichtung das Bilden einer Aerosolwolke, insbesondere einer Wolke, für die die Gesetze der Wolkenbewegung zumindest im Wesentlichen gelten. Die Vorrichtung ermöglicht weiterhin, dass diese Wolke, bevorzugt durch die Eigenbewegung der Wolke, insbesondere unter Nutzung der wolkenphysikalischen Bewegungsgesetze sowie der Gravitation, absinkt und es somit erlaubt, den Bodenbereich der Kammer, vorzugsweise die Positionierungsvorrichtung, und damit die Probe schnell, in der Regel in weniger als 3 min, gleichmäßig zu benetzen. Hier ermöglicht die Vorrichtung weiterhin das ungestörte Absinken der Wolke besonders ohne einen zugeführten oder generierten Luftstrom (luftstrom-frei bzw. luftfluss-frei), der beispielsweise die Bewegung des Aerosols beeinflusst und wie es beispielsweise im Stand der Technik der Fall ist. Vorteilhafterweise wird bereits das Aerosol luftstrom-frei erzeugt, also ohne Bereitstellung eines Luftstroms. Ein solcher Luftstrom wird im Stand der Technik beispielsweise zur Erzeugung des Aerosols beispielsweise mittels einer Düse oder zum Vorbeiführen des Aerosols an der Probe und damit einem teilweisen Abtransports des Aerosols eingesetzt. Bei luftstrom-freier Zuführung bleibt das gesamte erzeugte Aerosol in der Expositionskammer und kann somit sehr effizient durch die Gravitation und eine Anfangsgeschwindigkeit der Wolke in Richtung der Probe auf die Probe deponieren. Ein Beispiel für eine solche luftstrom-freie Aerosolisierungsvorrichtung sind im Stand der Technik bekannte Schwingmembranvernebler. Es ist bevorzugt, dass die Aerosolisierungsvorrichtung derart ausgestaltet ist, dass das austretende Aerosol eine gewisse Anfangsgeschwindigkeit in Richtung des Bodenbereichs hat, die von null verschieden ist.

Unter Wolke wird hierin vorzugsweise verstanden, wenn die Fallgeschwindigkeit des Aerosolensembles (unter dem Einfluss der Schwerkraft) mindestens um einen Faktor 10 höher ist, als die Fallgeschwindigkeit der Einzelaerosole (oder der verdünnten Wolke) und insbesondere auf Grund der Eigenbewegung der Wolke eine mittlere Fallgeschwindigkeit von größer als 1 cm/s, bevorzugt größer als 3 cm/s, besonders bevorzugt größer als 10 cm/s erreicht. In einer Wolke tritt somit vorzugsweise ein Windschatteneffekt auf: Zumindest einige der Aerosolpartikel, die im Inneren der Wolke sind, erfahren einen reduzierten Luftwiderstand, da sie im Windschatten der äußeren bzw. der sie umgebenden Aerosolpartikel fallen. Voraussetzung dafür ist insbesondere, dass die Wolke genügend dicht ist. Das Aerosolpartikelvolumen ist hierzu mindestens 10⁻⁴%, bevorzugt mindestens 10⁻³%, besonders bevorzugt 10⁻²% des Luftvolumens, in dem es enthalten ist.

Insbesondere kann die Vorrichtung gewährleisten, dass das Aerosol, vorzugsweise zumindest in den ersten 1 bis 10 Sekunden der Exposition, als wohl definierte, intakte Wolke oberhalb und direkt über dem Bodenbereich gebildet wird. Die Wolke kann dann ohne Störungen bspw. durch im Fallweg angebrachte Gegenstände oder durch, beispielsweise extern zugeführte, zusätzliche Luftströmungen in der Vorrichtung bzw. der durch sie gebildeten Expositionskammer absinken wobei es für den ungehinderten Fall der Wolke vorteilhaft ist, dass der Durchmesser der Wolke (senkrecht zur Fallrichtung der Wolke) deutlich kleiner als die lichte Weite der Kammer bei der jeweiligen Höhe ist, insbesondere dass die Querschnittsfläche der Expositionskammer zumindest in der oberen Hälfte der Kammer mehr als 2-fach, bevorzugt 2,5-fach, besonders bevorzugt mehr als 3-fach so groß wie die Querschnittsfläche der Wolke bei der jeweiligen Höhe ist.

Ein zusätzlicher bevorzugter Aspekt ist, dass die Wolke genügend Platz haben sollte, um nicht gestaut zu werden und damit ungehindert zu fallen. Dies kann beispielsweise und insbesondere dadurch gewährleistet werden, dass die Seitenwände der Kammer weit genug auseinander platziert sind.

Ein zusätzlicher bevorzugter Aspekt ist, dass die Wolke als integrale Struktur in der Nähe des Bodenbereichs ankommen sollte, um einen kräftigen Vortex zur gleichmäßigen räumlichen Verteilung des Aerosols in der Expositionskammer bilden zu können. Dies kann voraussetzen, dass die Fallhöhe nicht zu groß ist, da sich sonst die Wolke in der Näher des Bodenbereichs zu sehr verbreitert und aufgelöst hat. Falls die Fallhöhe bzw. der Abstand des Auslasses der Verneblungseinheit zum Bodenbereich zu gering ist, kann sich ebenfalls kein genügend großer Vortex bilden, um sich über den gesamten Bodenbereich zu erstrecken.

Bei der hier beschriebenen Vorrichtung unterliegen die freigesetzten Aerosolpartikel nach dem Verlassen der Aerosolisierungsvorrichtung nur noch der Wirkung der Massenträgheit und der Schwerkraft, d. h. dass keine weiteren externen Kräfte auf die Wolke einwirken.

Dies und entsprechende Merkmale der Vorrichtung erlauben insbesondere die Erzeugung einer wohl definierten Wolke mit einer starken "Eigenbewegung". Dafür sind vorzugsweise folgende Aspekte vorteilhaft: Der Durchmesser der Wolke, der Flüssigkeitsgehalt der Wolke und/oder die Geometrie der Expositionskammer, insbesondere deren Höhe sowie Länge/Breite. Diese Parameter zusammen bestimmen vorzugsweise die Fallgeschwindigkeit und strukturelle Integrität der Wolke in der Nähe des Bodenbereichs, die vorteilhaft sind für die Bildung eines genügend starken Vortex und damit die gleichmäßige Verteilung des Aerosols als Nebel in der Expositionskammer ist. Somit kann die mittlere Fallgeschwindigkeit der Wolke vorzugsweise als Ergebnis der strukturellen Eigenschaften der Vorrichtung benannt werden. Insbesondere wird die Fallgeschwindigkeit vorzugsweise immer geringer, je mehr die Expositionskammer mit Nebel gefüllt ist. Die hohen Fallgeschwindigkeiten ergeben sich also vorzugsweise nur am Beginn des Expositionsprozesses.

Insbesondere kann die hier beschriebene (Aerosol-)Wolke dadurch definiert sein, dass sie einen so hohen Flüssigkeitsgehalt hat, dass sie bei Tageslicht sichtbar ist. Im Unterschied zum Nebel ist eine Wolke vorzugsweise lokal begrenzt, d.h. sie ist deutlich abgegrenzt von einer Umgebung mit niedrigem Flüssigkeitsgehalt. Dadurch bewegt sich eine Wolke wie ein Gas mit höherer Dichte in einem Gas mit geringerer Dichte, d.h. eine Wolke entwickelt auf Grund der Gravitation eine starke Eigenbewegung und damit eine deutlich höhere Fallgeschwindigkeit als die Einzeltröpfchen sie erreichen würden, da die Tröpfchen im inneren der Wolke durch die äußeren Tröpfchen vor Luftwiderstand geschützt sind und somit schneller fallen können ("Windschatteneffekt", wie auch vorangehend angesprochen). Dieser Wolkeneffekt ist vorteilhaft für die hier vorgestellte Erfindung. Darüber hinaus wird eine Wolke, die bereits eine gewisse Anfangsgeschwindigkeit hat, nach den Gesetzen der Reibung abgebremst, wobei die Wolke als geometrische Gesamtheit für den Bewegungsablauf (nicht die Einzeltröpfchen) verantwortlich ist.

Für die gleichmäßige Verteilung des Aerosols auf dem Bodenbereich ist es bevorzugt und von Vorteil, wenn nicht die eng begrenze Wolke direkt auf dem Bodenbereich durch inertiale Impaktion deponiert, sondern die Wolke in einen gleichmäßigen Nebel überführt wird, der die gesamte Expositionskammer ausfüllt und der dann durch natürliche Sedimentation den Bodenbereich bzw. die dort angeordneten Positioniervorrichtung mit den darin befindlichen Proben gleichmäßig benetzt. Ein Nebel ist ebenfalls ein sehr dichtes Flüssigaerosol, das sich von einer Wolke darin unterscheidet, dass ein Nebel gleichmäßig verteilt und weit ausgedehnt ist, während eine Wolke räumlich begrenzt, nicht notwendigerweise gleichmäßig verteilt ist und einen scharfen Übergang zur umgebenden flüssigkeitsarmen Luft hat. Ein Nebel entsteht in der Vorrichtung beispielsweise und vorzugsweise durch die natürliche Verwirbelung der Wolke, wenn sich diese auf den Bodenbereich auftrifft und dort nach allen Seiten gleichmäßig abgelenkt wird, was zur Bildung von Vortices bzw. Wirbeln führt, die die scharf definierte Wolke in einen gleichmäßigen Nebel überführen.

Insbesondere kann der hier beschriebene Nebel als ein weit ausgedehntes, gleichmäßig verteiltes Flüssigaerosol beschrieben werden, dessen Flüssigkeitsgehalt so hoch ist, dass er auf Grund der Lichtstreuung an den Tropfen mit bloßem Auge sichtbar ist. Nebel ist räumlich sehr stabil; im Gegensatz zu einer Wolke entwickelt er keine starke Eigenbewegung.

Weiterhin ist es bevorzugt und von Vorteil, dass die verschiedenen Elemente der Vorrichtung geeignet, insbesondere im Verhältnis zueinander, dimensioniert sind. Beispielsweise kann die Aerosolisierungsvorrichtung insbesondere ihr Auslass ca. 5 cm bis 50 cm, weiter bevorzugt ca. 7 cm bis 30 cm und besonders bevorzugt ca. 9 cm bis 20 cm oberhalb des Bodenbereichs bzw. der Positionierungsvorrichtung und insbesondere der LFG angeordnet sein. Eine vertikale Seitenlänge bzw. Höhe der Seitenwände ist vorzugsweise circa 8 cm bis 53 cm, bevorzugt ca. 10 cm bis 33 cm und besonders bevorzugt ca. 12 cm bis 23 cm. Eine bevorzugte horizontale Seitenlänge bzw. Breite des Bodenbereichs und/oder der Seitenwände beträgt circa 5 cm bis 100 cm (insbesondere beim Einsatz mehrerer Vernebler, wie nachfolgend beschrieben) und vorzugsweise etwa 7 cm bis 50 cm und besonders bevorzugt etwa 8 cm bis 25 cm. Die Seitenwände können gleich lang sein, und einen in etwa quadratischen Bodenbereich und/oder Deckelbereich umfassen oder unterschiedliche Breiten aufweisen, und beispielsweise einen rechteckigen Bodenbereich und/oder Deckelbereich umfassen. Bei unterschiedlich langen Seitenwänden beträgt die andere bevorzugte horizontale Seitenlänge bzw. Tiefe des Bodenbereichs und/oder der Seitenwände circa 4 cm bis 80 cm (insbesondere beim Einsatz mehrerer Vernebler, wie nachfolgend beschrieben) und vorzugsweise etwa 5 cm bis 40 cm und besonders bevorzugt etwa 6 cm bis 20 cm. Das Verhältnis zwischen vertikaler Länge (Höhe) der Seitenwände zu der horizontalen Seitenlänge (Breite; Tiefe) beträgt in etwa 1/1 bis 2/1, und/oder weiter bevorzugt etwa 4/3 bis 1,5/1.

Ferner kann es besonders bevorzugt sein, dass der Quotient zwischen Fläche des Bodenbereichs zur vertikalen Ausdehnung der Seitenwände (jeweils gemessen in cm² bzw. cm) zwischen 3 und 12 vorzugsweise zwischen 4 und 9, besonders bevorzugt zwischen 5 und 7,5 beträgt (hier einheitslos aufgeführt).

Ebenso beträgt der Quotient zwischen Fläche des Bodenbereichs und Abstand des Einlasses der Aerosolisierungsvorrichtung zum Bodenbereich (jeweils gemessen in cm² bzw. cm) zwischen 3 und 12, besonders bevorzugt zwischen 4 und 9, weiter besonders bevorzugt zwischen 5 und 7.5 (hier einheitslos aufgeführt)

Eine derartige Dimensionierung erlaubt vorzugsweise eine vorteilhafte Aerosolwolkenbildung sowie eine effektive, gleichmäßige und schnelle Benetzung der Probe.

Für die Bildung eines gleichförmig verteilten Nebels in der gesamten Expositionskammer aus der Eigenbewegung der Wolke heraus ist es von Vorteil, dass die initiale Wolke (Wolke, die innerhalb der ersten Sekunden der Exposition gebildet wird, d.h. insbesondere innerhalb der ersten 10 Sekunden, bevorzugt innerhalb der ersten 5 Sekunden und besonders bevorzugt innerhalb der ersten 3 Sekunden der Wolkenbildung) als kompaktes Gebilde auf den Bodenbereich trifft, um genügend Impuls zu haben, um einen Vortex zu bilden, der sich gleichmäßig über den gesamten Bodenbereich ausbreitet. Dies bedingt vorzugsweise, dass der Einlass der Aerosolisierungsvorrichtung nicht mehr als ca. 50 cm, bevorzugt nicht mehr als 30 cm, besonders bevorzugt nicht mehr als 20 cm über dem Bodenbereich positioniert ist.

Insbesondere ist es bevorzugt und von Vorteil, wenn die Abstände zwischen gegenüberliegenden Seitenwänden so justiert sind, dass die Vortices die gesamte Fläche des dadurch definierten Bodenbereichs gleichmäßig ausfüllen. Dies beschränkt die bevorzugte maximale horizontale Ausdehnung der Expositionskammer vorzugsweise auf maximal ca. das 10-fache des Durchmessers der initialen Wolke am Eintrittspunkt in die Expositionskammer. Dieser initiale Durchmesser der Wolke kann beispielsweise ca. 2 cm betragen.

Gemäß einer bevorzugten Ausführungsform entspricht die Geometrie des Deckelbereichs im Wesentlichen dem Bodenbereich und ist vertikal dazu - in Schwerkraftrichtung oberhalb - angeordnet. Vorzugsweise erstreckt sich der Deckelbereich im Wesentlichen entlang der oder in der Nähe der durch die (oberen) Enden der Seitenwände aufgespannten imaginären Ebene und/oder wird durch die Seitenwände nach außen begrenzt. Entsprechend erstreckt sich der Bodenbereich im Wesentlichen entlang der oder in der Nähe der durch die (unteren) Enden der Seitenwände aufgespannten imaginären Ebene und/oder wird durch die Seitenwände nach außen begrenzt.

Weiterhin ist es bevorzugt, dass der Bodenbereich und/oder der Deckelbereich im Wesentlichen horizontal ist/sind. Dies wirkt sich vorzugsweise nicht nur vorteilhaft auf die Einfachheit der Konstruktion und Fertigung aus sondern auch auf die vor- und nachstehend beschriebene Funktonalität der Vorrichtung.

Während die Seitenwände, die wie beschrieben vorzugsweise den Boden- und/oder Deckelbereiche seitlich begrenzen, vorzugsweise im Wesentlichen senkrecht angeordnet sind, sind sie alternativ bevorzugt im Wesentlichen pyramidenförmig und/oder kegelförmig, sich nach unten öffnend bzw. erweiternd, ausgebildet.

Die hier diskutierten Dimensionen und Parameter der Vorrichtung sind vorzugsweise derart ausgebildet und vorzugsweise derart aufeinander abgestimmt, dass die beschriebenen Funktionalitäten gewahrt und/oder ermöglicht werden. Beispielsweise ist es von Vorteil, dass die Aerosolisierungsvorrichtung ein Aerosol derart erzeugt, dass dieses eine Wolke bildet. Diese durch die Aerosolisierungsvorrichtung gebildete Aerosolwolke kann vorzugsweise im Wesentlichen frei fallen, insbesondere damit die daraus resultierende starke Eigenbewegung der Wolke beim Auftreffen auf den Bodenbereich zur Bildung eines stark ausgeprägten Vortex führt. Hierzu ist es vorteilhaft, dass die Aerosolisierungsvorrichtung, die Wolke und/oder die Vorrichtung derart ausgebildet ist/sind, dass die Querschnittsfläche der Wolke (senkrecht zur Fallrichtung der Wolke) deutlich kleiner als die lichte Weite der Kammer bei der jeweiligen Höhe ist, insbesondere dass die Querschnittsfläche der Expositionskammer zumindest in der oberen Hälfte der Kammer mehr als 2-fach, bevorzugt 2,5-fach, besonders bevorzugt mehr als 3-fach so groß wie die Querschnittsfläche der Wolke bei der jeweiligen Höhe beträgt.

Der Deckelbereich kann, abgesehen von den jeweiligen Endabschnitten der Seitenwände, lediglich als eine Öffnung oder Aussparung ausgebildet sein. Es ist jedoch besonders bevorzugt, dass der Deckelbereich eine Deckelwand aufweist. Dies kann den Bereich, der durch die vertikalen Seitenwände von der Umgebung abgetrennt ist, und auch als Expositionskammer bezeichnet wird, weiter von der Umgebung abschirmen.

Die Deckelwand kann abnehmbar ausgestaltet bzw. abnehmbar an den Seitenwänden angeordnet sein. Alternativ kann diese integral mit den Seitenwänden ausgebildet sein. Besonders bevorzugt kann die Deckelwand auch eine Aussparung bzw. eine Öffnung aufweisen oder einen Bereich aufweisen, der abnehmbar oder zum Öffnen ausgestaltet ist. Dieser abnehmbare Bereich bzw. diese Öffnung der Deckelwand ist besonders bevorzugt derart ausgestaltet bzw. dimensioniert, dass die Aerosolisierungsvorrichtung das Aerosol durch diesen bzw. durch diese in die Expositionskammer vertikal nach unten in Richtung Bodenbereich einleiten kann. Die Aerosolisierungsvorrichtung kann dazu vertikal knapp oberhalb des Deckelbereichs, genau auf Höhe des Deckelbereichs oder vertikal knapp unterhalb des Deckelbereichs angeordnet werden. Die Position der Aerosolisierungsvorrichtung bestimmt sich dabei vorzugsweise durch ihren Aerosolauslass, vorzugsweise durch den Ort der Aerosolerzeugung, bspw. der Schwingmembran. Die Positionierung der Aerosolisierungsvorrichtung kann zu einer besonders geeigneten und vorteilhaften Wolkenbildung führen.

Weiterhin ist es besonders bevorzugt, dass die Seitenwände zumindest teilweise durchsichtig sind. Beispielsweise können die Seitenwände Glas und/oder Kunststoff, beispielsweise ein durchsichtiges Polykarbonat, vorzugsweise Makrolon, aufweisen oder daraus bestehen. Letzteres ist insbesondere vorteilhaft, da es sich leicht mit bspw. Alkohol sterilisieren lässt. Darüber hinaus kann es dem Benutzer möglich sein, Phänomene, die sich innerhalb der Vorrichtung abspielen, zu beobachten. Insbesondere kann der Benutzer hierdurch in die Lage versetzt werden zu beobachten, in wieweit eine Wolken- und Vortexbildung und/oder Benetzung abgeschlossen bzw. in wieweit dies fortgeschritten ist. Dies dient bevorzugt der Qualitätskontrolle und der Optimierung der Vorgehensweise.

Weiterhin kann die Vorrichtung hierdurch relativ leicht (gewichtsarm) ausgestaltet sein - beispielsweise kann die Vorrichtung derart ausgestaltet sein, dass ihr Gesamtgewicht (bspw. ohne Positionierungsvorrichtung) nicht mehr als fünf Kilogramm, vorzugsweise nicht mehr als ein Kilogramm, beispielsweise circa 800 Gramm beträgt (vorzugsweise umfasst die Positionierungsvorrichtung dabei keine zusätzlichen, gewichtsrelevanten Merkmale wie beispielsweise eine Heizeinrichtung). Durch eine leichte Ausgestaltung und weiter durch die vorstehend beschrieben geeignete Dimensionierung kann eine solche Vorrichtung insbesondere auch zur Verwendung in einer Sterilbank bzw. Sterilkammer genutzt werden.

Vorzugsweise weist die Deckelwand zumindest eines der Materialien auf, das auch die Seitenwände aufweisen. Dies kann beispielsweise fertigungstechnisch besonders einfach zu realisieren sein. Weiter führt dies zu einer einheitlichen Erscheinung der Vorrichtung.

Vorzugsweise ist die Vorrichtung und insbesondere ihre Komponenten, beispielsweise die Seitenwände, nach außen luft- bzw. gasdicht ausgestaltet. Eine solche Vorrichtung eignet sich besonders gut auch für toxikologische Experimente bzw. zur Untersuchung von toxikologischen Substanzen. Insbesondere können in einer solchen Ausgestaltung auch die Komponenten (beispielsweise die Seitenwände) gegeneinander entsprechend luft- bzw. gasdicht abgedichtet sein, beispielsweise durch die Verwendung von Dichtungsmaterialien.

Die Aerosolisierungsvorrichtung ist vorzugsweise als ein Vernebler ausgestaltet. Weiter kann die Aerosolisierungsvorrichtung aber auch mehrere Vernebler aufweisen, die geeignet zueinander angeordnet sind. Dabei können die Vernebler vorzugsweise nah bzw. eng beieinander angeordnet werden, insbesondere so dass die durch die Vernebler generierten jeweiligen Aerosolwolken schon kurz nach der Generierung zu einer großen Wolke verschmelzen. Alternativ und/oder zusätzlich können die Vernebler der Aerosolisierungsvorrichtung vorzugsweise so weit voneinander entfernt angeordnet werden, dass jeder Vernebler eine separate Wolke generiert, wobei die einzelnen Aerosolwolken erst nach dem Absinken und Verwirbeln zu einem Nebel verschmelzen, der eine gleichmäßige Tröpfchendeposition erlaubt. Hierbei ist insbesondere die räumliche Anordnung der Vernebler von Bedeutung, die wiederum von der Geometrie der Kammer, der aktiven Querschnittsfläche (Durchmesser) der initialen Wolke und der Flüssigkeitsausstoßrate des Verneblers abhängen kann.

Die bevorzugten Schwingmembranvernebler können beispielsweise eine Membran aufweisen, die vorzugsweise parallel zur Horizontalen ausgerichtet ist. Eine derart ausgestaltete Aerosolisierungsvorrichtung kann insbesondere eine geeignete Wolkenbildung, wie vorstehend beschrieben, gewährleisten.

Insbesondere ist die Aerosolisierungsvorrichtung vorzugsweise angepasst, Aerosolpartikel mit einer mittleren Größe zwischen etwa 1 µm und 15 µm, bevorzugt zwischen etwa 2 µm und 10 µm und besonders bevorzugt zwischen etwa 3 µm und 7 µm zu erzeugen bzw. freizusetzen. Auch dies beeinflusst eine geeignete Wolkenbildung und die Sedimentationszeit des Nebels vorzugsweise weiter positiv. Zu große Tropfen impaktieren direkt bei der Annäherung der initialen Wolke an den Bodenbereich. Da die initiale Wolke nicht gleichmäßig über den Bodenbereich verteilt ist, würde dies zu ungleichförmiger Benetzung des Bodenbereichs führen. Zu kleine Tropfen haben nicht genug Masse, um effizient auf die Bodenplatte zu sedimentieren, was die Sedimentationszeit und damit die Zeit für eine Exposition verlängern und die Depositionseffizienz reduzieren würde.

Vorzugsweise ist die Aerosolisierungsvorrichtung, insbesondere in einer vertikalen Draufsicht, mittig über dem Bodenbereich bzw. der Positionierungsvorrichtung angeordnet. Dabei fällt der Mittelpunkt des Bodenbereichs bzw. der Positionierungsvorrichtung in einer vertikalen Draufsicht vorzugsweise in etwa mit dem Mittelpunkt der Aerosolisierungsvorrichtung zusammen. Umfasst die Aerosolisierungsvorrichtung mehrere Vernebler, sind diese dabei vorzugsweise im Wesentlichen symmetrisch zum Mittelpunkt des Bodenbereichs bzw. der Positioniervorrichtung angeordnet Dies kann insbesondere begünstigen, dass die Wolkenbildung symmetrisch und gleichmäßig erfolgt und die Probe daher besonders gleichmäßig mit der Substanz benetzt wird.

Gemäß einer weiteren bevorzugten Ausführungsform weist die Vorrichtung eine Lichtquelle auf. Diese ist vorzugsweise dazu angepasst bzw. angeordnet, einem Benutzer anzuzeigen, ob die Wolke in der Expositionskammer bereits vollständig abgesunken ist. Beispielsweise kann es sich bei der Lichtquelle um eine Laserquelle handeln, beispielsweise einen "Laserpointer". Die Lichtquelle ist vorzugsweise derart angeordnet ist, dass ein definierter Lichtstrahl in die Kammer emittiert wird. Vorzugsweise wird ein Strahl mit einem Durchmesser erzeugt, der kleiner als 1 cm ist. Vorzugsweise ist die Anordnung derart, dass der emittierte Laserstrahl im Wesentlichen senkrecht zu einer Blickrichtung eines Benutzers verläuft. Hierdurch kann der Benutzer einerseits vor der Laserstrahlung geschützt werden. Andererseits nimmt der Benutzer den Laserstrahl als solchen nur wahr, wenn er gestreut wird - beispielsweise an feinen Partikeln in einer Wolke oder in einem Nebel. Durch eine derartige Lichtquelle wird also eine einfache und sichere Möglichkeit bereitgestellt, zu beobachten, ob die Wolke bzw. der Nebel noch vorhanden sind oder ob sich das Aerosol bereits auf die Probe abgesetzt hat. In anderen Worten ist ein derartiger Lichtstrahl für den Betreiber der Vorrichtung (der beispielsweise von "vorne" in die Kammer hineinblickt) unsichtbar, es sei denn, die Kammer enthält eine genügend dichte Wolke bzw. einen genügend dichten Nebel, an deren Tröpfchen das Licht in alle Richtungen, also auch nach vorne, gestreut wird. Sobald der Lichtstrahl nicht mehr sichtbar ist, ist die Wolke vollständig auf den Bodenbereich abgesunken und die Benetzung der Probe kann als abgeschlossen betrachtet werden. Vorzugsweise ist eine solche Lichtquelle an einer Seitenwand derart angeordnet, dass der Strahl senkrecht durch die Seitenwand verläuft, besonders bevorzugt in einer horizontal zentrierten Position der Seitenwand. Vertikal ist es besonders bevorzugt, dass die Lichtquelle in einem Abstand zwischen ca. 5 und 8 cm oberhalb der Positionierungsvorrichtung angeordnet ist. Insgesamt kann hierdurch einfache und benutzerfreundliche Kontrolle bereitgestellt werden, ob die Wolke in der Kammer bereits vollständig auf den Bodenbereich abgesunken und damit der Benetzungsvorgang beendet ist, die im Vergleich zur rein visuellen Kontrolle vorteilhaft ist.

Weiter ist es bevorzugt, dass die Vorrichtung weiter eine Kontrollkammer aufweist, die beispielsweise durch mindestens eine Wand von der Expositionskammer getrennt ist derart, dass das von der Aerosolisierungsvorrichtung erzeugte Aerosol nicht in die Kontrollkammer gelangt. Hierdurch können neben den Proben, die von dem Aerosol benetzt werden, auch Proben vorgesehen werden, die eine solche Benetzung nicht erfahren, ansonsten aber den gleichen Einflüssen ausgesetzt sind. Derartige Proben können für Vergleichszwecke oder als Kontrollen insbesondere für biologische Proben genutzt werden, um sicher zu stellen, dass die Handhabung von biologischen Proben selbst keine (unbeabsichtigte) biologische Wirkung auf die Proben hat.

Gemäß einem weiteren Aspekt betrifft die Erfindung auch ein Verfahren zur Benetzung mindestens einer Probe mit einem Aerosol. Insbesondere kann dieses Verfahren mit einer Vorrichtung wie vorstehend beschrieben ausgeführt werden. Es umfasst ferner vorzugsweise die im Zusammenhang mit der obigen Diskussion der Vorrichtung beschriebenen Schritte bzw. Funktionalitäten.

Das Verfahren umfasst vorzugsweise die Schritte Bereitstellen mindestens einer Probe, Erzeugen und Bereitstellen einer Aerosolwolke oberhalb, vorzugsweise senkrecht oberhalb, der mindestens einen Probe und Absinken der Aerosolwolke in Richtung der Probe.

Vorzugsweise bildet die Wolke bei Annäherung an den Bodenbereich mindestens einen Vortex und wird in einen Nebel konvertiert. Dieser sinkt dann in Richtung der Probe.

Die Aerosolpartikel werden dabei durch die luftstrom-freie Aerosolisierungsvorrichtung mit einer gewissen Austrittsgeschwindigkeit (oder Anfangsgeschwindigkeit) vorzugsweise in Richtung der Probe bzw. der Positionierungsvorrichtung freigesetzt.

Die Aerosolwolke sinkt ab, vorzugsweise gravitationsbedingt und aufgrund der Austrittsgeschwindigkeit der Wolke aus dem Vernebler. Die Wolke fällt somit vorzugsweise frei. Anschließend wird die Wolke vorzugsweise im Bodenbereich abgelenkt, beispielsweise an der Probe und/oder der Positionierungsvorrichtung. Die Ablenkung erfolgt vorzugsweise gleichmäßig seitlich in alle Richtungen und es erfolgt dann eine neuerliche Richtungsänderung der Wolke nach oben durch Ablenkung an den Seitenwänden. Dadurch entstehen, vorzugsweise gleichmäßige, Vortices bzw. Wirbel, die insbesondere zu einer gleichmäßigen Verteilung der Aerosolwolke und damit zur Bildung eines Aerosolnebels, insbesondere im unteren Teil der Expositionskammer, führen. Insbesondere bei fortschreitender Verneblung füllt sich somit die Vorrichtung allmählich von unten nach oben mit Nebel. Dieser Nebel ist gravitationsbedingt unten bzw. in Bodennähe am dichtesten. Anschließend, insbesondere nach Beendigung der Verneblung, sinkt der Nebel auf die Positionierungsvorrichtung und die darauf angeordnete mindestens eine Probe. Die Proben werden, unabhängig vom Ort ihrer Anordnung auf der Positionierungsvorrichtung gleichmäßig benetzt. Die Dauer vom Beginn der Vernebelung an bis hin zum Abschluss des, insbesondere gleichmäßigen, Benetzens beträgt vorzugsweise weniger als 12 min, bevorzugt weniger als 6 min, besonders bevorzugt weniger als 3 min.

Die Proben sind, wie eingangs beschrieben, Kulturzellen, vorzugsweise Epithelzellen, die vorzugsweise an der LFG angeordnet bzw. kultiviert sind. Ihre Anordnung erfolgt insbesondere auf einer Standardmikrotiterplatte. Ebenso kann es sich aber auch um anderes biologisches Material, beispielsweise Gewebe, Bakterien oder Ähnliches handeln. Auch eine Realisierung der Proben als Werkstoffproben ist möglich.

Vorzugsweise weist das Aerosol und damit die Wolke bzw. der Nebel eine pharmazeutisch und/oder toxikologisch wirksame Substanz auf.

Vorzugsweise weist das Aerosol eine flüssige Substanz auf, die beispielsweise in die Aerosolisierungsvorrichtung einpipettiert wird. Die generierte Aerosolwolke ist vorzugsweise derart, insbesondere so dicht (hoher Flüssigkeitsgehalt), dass die Gesetze der Wolkenbewegung gelten, wobei bevorzugt die oben beschriebenen Parameter für die Wolkeneigenschaften gelten. Dies führt insbesondere dazu, dass die Wolke durch ihre Austrittsgeschwindigkeit aus dem Vernebler und die Gravitationskraft (d.h. durch Fallen) absinkt, bei Annäherung an den Bodenbereich Vortices bildet, die die Wolke in einen Nebel überführen, der dann durch Gravitation gleichmäßig auf den Bodenbereich und die dort befindliche Probe absinkt.

Dem Fachmann wird klar sein, dass die vorstehend beschriebenen Aspekte, die ausführlich im Zusammenhang mit der erfindungsgemäßen Vorrichtung beschrieben wurden, auch in dem erfindungsgemäßen Verfahren realisiert werden.

Im Rahmen dieser Beschreibung werden zusätzlich die folgenden Aspekte im Zusammenhang mit der Erfindung offenbart:
1. Vorrichtung (2) zur Benetzung mindestens einer Probe mittels eines Aerosols,
   wobei die Vorrichtung (2) aufweist Seitenwände (4a, 4b, 4c, 4d),
   einen Bodenbereich (6), der durch die Seitenwände (4a, 4b, 4c, 4d) begrenzt ist,
   wobei der Bodenbereich (6) zur Aufnahme von oder zum Zusammenwirken mit einer Positionierungsvorrichtung (8) zur Positionierung der Probe(n), die beispielsweise in Aushebungen (18) platziert ist/sind, angepasst ist,
   einem Deckelbereich (10), der dem Bodenbereich (6) vertikal gegenüberliegt, und der durch die Seitenwände (4a, 4b, 4c, 4d) begrenzt ist,
   wobei die Vorrichtung (2) eine Expositionskammer (12) bildet,
   eine Aerosolisierungsvorrichtung (14) zur Erzeugung eines Aerosols (16), die in der Nähe des Deckelbereichs (10) angeordnet ist,
   wobei die Aerosolisierungsvorrichtung (14) über dem Bodenbereich (6) angeordnet ist, derart dass die Aerosolisierungsvorrichtung (14) den Bodenbereich (6) in einer vertikalen Draufsicht zumindest teilweise überdeckt.
2. Vorrichtung (2) zur Benetzung mindestens einer Probe mittels eines Aerosols,
   wobei die Vorrichtung (2) aufweist Seitenwände (4a, 4b, 4c, 4d),
   einen Bodenbereich (6), der durch die Seitenwände (4a, 4b, 4c, 4d) begrenzt ist,
   wobei der Bodenbereich (6) zur Aufnahme von oder zum Zusammenwirken mit einer Positionierungsvorrichtung (8) zur Positionierung der Probe angepasst ist,
   einem Deckelbereich (10), der dem Bodenbereich (6) vertikal gegenüberliegt, und der durch die Seitenwände (4a, 4b, 4c, 4d) begrenzt ist,
   wobei die Vorrichtung (2) eine Expositionskammer (12) bildet,
   eine Aerosolisierungsvorrichtung (14) zur Erzeugung eines Aerosols (16), die vorzugsweise in der Nähe des Deckelbereichs (10) angeordnet ist,
   wobei die Vorrichtung (2) und/oder die Aerosolisierungsvorrichtung (14) ausgebildet sind, eine Aerosolwolke zu bilden.
3. Vorrichtung (2) zur Benetzung mindestens einer Probe mittels eines Aerosols,
   wobei die Vorrichtung (2) aufweist Seitenwände (4a, 4b, 4c, 4d),
   einen Bodenbereich (6), der durch die Seitenwände (4a, 4b, 4c, 4d) begrenzt ist,
   wobei der Bodenbereich (6) zur Aufnahme von oder zum Zusammenwirken mit einer Positionierungsvorrichtung (8) zur Positionierung der Probe(n), die beispielsweise in Aushebungen (18) platziert ist/sind, angepasst ist,
   einem Deckelbereich (10), der dem Bodenbereich (6) vertikal gegenüberliegt, und der durch die Seitenwände (4a, 4b, 4c, 4d) begrenzt ist,
   wobei die Vorrichtung (2) eine Expositionskammer (12) bildet,
   eine Aerosolisierungsvorrichtung (14) zur Erzeugung eines Aerosols (16), die vorzugsweise in der Nähe des Deckelbereichs (10) angeordnet ist,
   wobei die Aerosolisierungsvorrichtung als luftstrom-freie Aerosolisierungsvorrichtung ausgestaltetet ist.
4. Vorrichtung (2) nach einem der vorhergehenden Aspekte, wobei die Vorrichtung keine Aerosolisierungsvorrichtung aufweist sondern ausgebildet ist, eine solche aufzunehmen oder mit einer solchen zusammenzuwirken.
5. Vorrichtung (2) nach einem der vorhergehenden Aspekte, wobei die Aerosolisierungsvorrichtung (14) über dem Bodenbereich (6) angeordnet ist, derart dass die Aerosolisierungsvorrichtung (14) den Bodenbereich (6) in einer vertikalen Draufsicht zumindest teilweise überdeckt.
6. Vorrichtung (2) nach einem der vorhergehenden Aspekte, wobei die Seitenwände (4a, 4b, 4c, 4d) im Wesentlichen vertikal sind, und/oder
   wobei der Bodenbereich (6) im Wesentlichen horizontal ist, und/oder
   wobei der Bodenbereich (6) durch die Seitenwänden (4a, 4b, 4c, 4d) seitlich begrenzt ist, und/oder
   wobei der Deckelbereich (10) durch die Seitenwände (4a, 4b, 4c, 4d) seitlich begrenzt ist.
7. Vorrichtung (2) nach einem der vorhergehenden Aspekte, wobei die Aerosolisierungsvorrichtung (14) angepasst ist, das Aerosol in Richtung (R) des Bodenbereichs (6) freizusetzen.
8. Vorrichtung (2) nach einem der vorhergehenden Aspekte, wobei die Aerosolisierungsvorrichtung (14) in vertikaler Draufsicht innerhalb der bzw. zwischen den Seitenwände(n) (4) angeordnet ist und vorzugsweise einen geeigneten Mindestabstand zu den Seitenwänden aufweist, der vorzugsweise mindestens 3 cm beträgt.
9. Vorrichtung (2) nach einem der vorhergehenden Aspekte, wobei das Aerosol eine Flüssigkeit und/oder einen Feststoff aufweist und vorzugsweise flüssige und/oder feste Schwebeteilchen und ein Gas umfasst.
10. Vorrichtung (2) nach einem der vorhergehenden Aspekte, wobei die Vorrichtung (2) und/oder die Verneblungsvorrichtung (14) ausgebildet sind, eine Wolke zu bilden.
11. Vorrichtung (2) nach einem der vorhergehenden Aspekte, wobei die Vorrichtung eingerichtet ist, ohne externen Luftstrom, und insbesondere luftstrom-frei, betrieben zu werden.
12. Vorrichtung (2) nach einem der vorhergehenden Aspekte, wobei die Aerosolisierungsvorrichtung mindestens einen Vernebler umfasst und vorzugsweise einen als luftstrom-freien Vernebler ausgestalteten Vernebler aufweist und vorzugsweise einen Schwingmembranvernebler aufweist und vorzugsweise ein solcher ist.
13. Vorrichtung (2) nach einem der vorhergehenden Aspekte, wobei die Aerosolisierungsvorrichtung (14) so orientiert ist, dass die initiale Bewegungsrichtung der Wolke mit der Vertikalen einen Winkel kleiner 30° einschließt und bevorzugt im Wesentlichen parallel zur Vertikalen ausgerichtet ist und die Aerosolisierungsvorrichtung (14) bevorzugt eine perforierte Membran aufweist, die mit der Horizontalen einen Winkel kleiner 30° einschließt und die bevorzugt im Wesentlichen parallel zur Horizontalen ausgerichtet ist.
14. Vorrichtung (2) nach einem der vorhergehenden Aspekte, wobei in einer vertikalen Draufsicht die Aerosolisierungsvorrichtung in etwa mittig über dem Bodenbereich bzw. der Positionierungsvorrichtung angeordnet ist.
15. Vorrichtung (2) nach einem der vorhergehenden Aspekte, wobei die Vorrichtung derart ausgebildet ist, dass eine von der Vernebelungsvorrichtung initial erzeugte Wolke so beschaffen ist, dass die mittlere Fallgeschwindigkeit der am Anfang der Exposition erzeugten Wolke größer als 1 cm/s, bevorzugt größer als 3 cm/s, besonders bevorzugt größer als 10 cm/s ist, wobei diese Geschwindigkeit vorzugsweise die Austrittsgeschwindigkeit der Wolke aus der Aerosolisierungsvorrichtung ist.
16. Vorrichtung (2) nach einem der vorhergehenden Ansprüche, wobei die Vorrichtung derart ausgebildet ist, dass die von der Aerosolisierungsvorrichtung erzeugte Wolke so beschaffen ist, dass die Wolke unbehindert fallen kann und somit ein rascher Transport des Aerosols zum Bodenbereich gewährleistet wird.
17. Vorrichtung (2) nach einem der vorhergehenden Ansprüche, wobei die Expositionskammer derart dimensioniert ist, dass die horizontale Querschnittsfläche der Expositionskammer in der oberen Hälfte der Kammer mehr als 2-fach, bevorzugt 2,5-fach, besonders bevorzugt mehr als 3-fach so groß wie die Querschnittsfläche der Wolke ist.
18. Vorrichtung (2) nach einem der vorhergehenden Aspekte, mit einer Positionierungsvorrichtung (8) zur Positionierung einer Probe.
19. Vorrichtung (2) nach einem der vorhergehenden Aspekte, wobei die Vorrichtung und/oder die Positionierungsvorrichtung ausgebildet ist/sind, als Probe vorzugsweise Zellen und besonders vorzugsweise Epithelzellen beispielsweise in Aushebungen (18) aufzunehmen bzw. zu benetzen.
20. Vorrichtung (2) nach einem der vorhergehenden Aspekte, wobei die Vorrichtung und/oder die Positionierungsvorrichtung derart angepasst ist/sind, dass die Probe als Luft-Flüssigkeits-Grenzschicht (LFG) angeordnet werden kann.
21. Vorrichtung (2) nach einem der vorhergehenden Aspekte, wobei die Positionierungsvorrichtung Elemente zur Messung und/oder Regulierung einer Temperatur, beispielsweise eine elektrische Heizung oder eine mit Hilfe eines Wasserkreislaufes betriebene Temperiervorrichtung, Elemente zur Versorgung der Probe, bspw. mit Nährmedium, und/oder Elemente zum Messen des Gewichts auf einer oder mehreren Proben deponierten Substanz, beispielsweise eine oder mehrere Quartzkristall-Mikrowaagen, aufweist.
22. Vorrichtung nach einem der vorhergehenden Aspekte, wobei die Aerosolisierungsvorrichtung, insbesondere ihr Auslass, vertikal zwischen 5 cm und 50 cm oberhalb des Bodenbereichs bzw. der Positionierungsvorrichtung angeordnet ist, bevorzugt zwischen 7 cm und 30 cm, besonders bevorzugt zwischen 9 cm und 20 cm und insbesondere ca. 16 cm.
23. Vorrichtung nach einem der vorhergehenden Aspekte, wobei die Aerosolisierungsvorrichtung, vorzugsweise die Schwingmembran eines Verneblers, vertikal oberhalb des Deckelbereichs angeordnet ist, vorzugsweise zwischen 0 cm und 10 cm, besonders bevorzugt zwischen 3 cm und 7 cm und insbesondere ca. 5 cm darüber.
24. Vorrichtung nach einem der vorhergehenden Aspekte, wobei die Aerosolisierungsvorrichtung vertikal unterhalb des Deckelbereichs angeordnet ist, vorzugsweise zwischen 0 cm und 10 cm, besonders bevorzugt zwischen 3 cm und 7 cm und insbesondere ca. 5 cm darunter.
25. Vorrichtung nach einem der vorhergehenden Aspekte, wobei die Seitenwände zumindest teilweise durchsichtig sind.
26. Vorrichtung nach einem der vorhergehenden Aspekte, wobei die Seitenwände Glas und/oder einen Kunststoff, zum Beispiel Polycarbonat aufweisen und besonders bevorzugt zumindest Bereiche der Seitenwände ein solches Material aufweisen oder aus einem solchen Material bestehen.
27. Vorrichtung nach einem der vorhergehenden Aspekte, wobei die Seitenwände eine vertikale Ausdehnung zwischen 8 cm und 53 cm, vorzugsweise zwischen 10 cm und 33 cm, besonders bevorzugt zwischen 12 cm und 23 cm und insbesondere ca. 19 cm haben.
28. Vorrichtung nach einem der vorhergehenden Aspekte, wobei der Bodenbereich eine erste horizontale Seitenlänge zwischen 5 cm und 100 cm, bevorzugt zwischen 7 cm und 50 cm, besonders bevorzugt zwischen 8 cm und 25 cm und eine zweite horizontale Seitenlänge zwischen 4 cm und 80 cm, bevorzugt zwischen 5 und 40 cm, besonders bevorzugt zwischen 6 cm und 20 cm aufweist.
29. Vorrichtung nach einem der vorhergehenden Aspekte, wobei der Bodenbereich genau zur Aufnahme einer Positionierungsvorrichtung, umfassend eine, zwei, drei oder vier Mikrotiterplatten ausgebildet ist.
30. Vorrichtung nach einem der vorhergehenden Aspekte, wobei die Vorrichtung zur gleichmäßigen Benetzung mehrerer, vorzugsweise von 6, 12, 24, 48, 96, 384 oder 1536 Proben, bzw. 2 x (6, 12, 24, 48, 96, 384 oder 1536) oder 3 x (6, 12, 24, 48, 96, 384 oder 1536) oder 4 x (6, 12, 24, 48, 96, 384 oder 1536) Proben, ausgebildet ist.
31. Vorrichtung nach einem der vorhergehenden Aspekte, wobei der Deckelbereich und/oder die Vorrichtung im Deckelbereich eine Deckelwand aufweist, die sich vorzugsweise im Wesentlichen horizontal erstreckt.
32. Vorrichtung nach einem der vorhergehenden Aspekte, wobei die Deckelwand abnehmbar an den vertikalen Seitenwänden angeordnet oder mit diesen verbunden ist, und/oder eine Öffnung bzw. eine Aussparung aufweist und/oder einen Bereich aufweist, der ausgestaltet ist geöffnet zu werden.
33. Vorrichtung nach einem der vorhergehenden Aspekte, wobei der abnehmbare Bereich bzw. die Öffnung derart ausgestaltet bzw. dimensioniert ist, dass die Vorrichtung inwändig gereinigt und die Probe(n) auf dem Bodenbereich platziert werden können.
34. Vorrichtung nach Aspekt 32 oder 33, wobei ein weiterer abnehmbarer Bereich oder eine Öffnung im Wesentlichen mittig im Deckelbereich angeordnet ist.
35. Vorrichtung nach einem der vorhergehenden Aspekte, wobei der weitere abnehmbare Bereich bzw. die Öffnung derart ausgestaltet bzw. dimensioniert ist, dass die Aerosolisierungsvorrichtung das Aerosol dadurch in die Expositionskammer einleiten kann.
36. Vorrichtung nach einem der vorhergehenden Aspekte, wobei die Deckelwand zumindest eines der Materialen aufweist, das die Seitenwände aufweisen.
37. Vorrichtung nach einem der vorhergehenden Aspekte, wobei der vertikale Abstand der Aerosolisierungsvorrichtung, insbesondere von deren Auslass, von der Probe und vorzugsweise von der durch eine biologische Probe repräsentierten Luft-Flüssigkeits-Grenzschicht zwischen 5 cm und 50 cm, vorzugsweise zwischen 7 cm und 30 cm und besonders bevorzugt zwischen 9 cm und 20 cm, insbesondere ca. 16 cm beträgt.
38. Vorrichtung nach einem der vorhergehenden Aspekte, wobei die Vorrichtung genau einen Vernebler aufweist.
39. Vorrichtung nach einem der vorhergehenden Aspekte, wobei die Vorrichtung mehrere Vernebler aufweist.
40. Vorrichtung nach einem der vorhergehenden Aspekte, wobei die Aerosolisierungsvorrichtung angepasst ist, Aerosolpartikel mit einem mittleren aeerodynamischen Durchmesser zwischen 1 um und 15 µm, bevorzugt zwischen 2 µm und 10 µm, besonders bevorzugt zwischen 3 µm und 7 µm zu erzeugen und/oder freizusetzen.
41. Vorrichtung nach einem der vorhergehenden Aspekte und zusätzlich mit einer Laserquelle, die derart ausgebildet und positioniert ist, einen Laserstrahl im Wesentlichen parallel zur Horizontalen und senkrecht zur Blickrichtung des Operators durch mindestens eine Seitenwand zu emittieren, sodass der Laserstrahl für einen Benutzer genau dann sichtbar ist, wenn sich eine Wolke bzw. ein Nebel in der Expositionskammer befindet.
42. Vorrichtung nach einem der vorhergehenden Aspekte, wobei die Vorrichtung weiter eine Kontrollkammer aufweist, die, vorzugsweise durch mindestens eine Wand, von der Expositionskammer getrennt ist, derart, dass das von der Aerosolisierungsvorrichtung erzeugte Aerosol nicht in die Kontrollkammer gelangt.
43. Verfahren zur Benetzung mindestens einer Probe mittels eines Aerosols, insbesondere mit einer Vorrichtung nach einem der vorhergehenden Aspekte, wobei das Verfahren die Schritte aufweist
   Bereitstellen mindestens einer Probe,
   Erzeugen einer Aerosolwolke (16) Oberhalb, vorzugsweise senkrecht oberhalb, der mindestens einen Probe an einer Position, die den Bodenbereich (6) in einer vertikalen Draufsicht zumindest teilweise überdeckt
   und Absinkenlassen der Aerosolwolke in Richtung (R) der Probe.
44. Verfahren zur Benetzung mindestens einer Probe mittels eines Aerosols, insbesondere mit einer Vorrichtung nach einem der vorhergehenden Aspekte, wobei das Verfahren die Schritte aufweist
   Bereitstellen mindestens einer Probe,
   Erzeugen einer Aerosolwolke (16)
   und Absinkenlassen der Aerosolwolke in Richtung (R) der Probe.
45. Verfahren zur Benetzung mindestens einer Probe mittels eines Aerosols, insbesondere mit einer Vorrichtung nach einem der vorhergehenden Aspekte, wobei das Verfahren die Schritte aufweist
   Bereitstellen mindestens einer Probe,
   Erzeugen eines Aerosols (16) mittels eines luftstrom-freien Verneblers,
   und Absinkenlassen des Aerosols in Richtung (R) der Probe.
46. Verfahren nach einem der Aspekte 44 bis 46, ferner mit dem Schritt Ausformung von Vortices, die die Wolke in einen Nebel überführen, der die Expositionskammer in vertikaler Draufsicht auf den Bodenbereich gleichförmig ausfüllt.
47. Verfahren nach einem der Aspekte 44 bis 47, ferner mit dem Schritt gravitationsbedingtes Absinken des Nebels.
48. Verfahren nach einem der Aspekte 45 bis 48, wobei der Schritt des Erzeugens des Aerosols das Erzeugen einer Aerosolwolke umfasst.
49. Verfahren nach einem der Aspekte 44 bis 49, wobei der Schritt des Erzeugens des Aerosols (16) bzw. der Schritt des Erzeugens der Aerosolwolke vertikal oberhalb der mindestens einen Probe an einer Position erfolgt, die den Bodenbereich (6) in einer vertikalen Draufsicht zumindest teilweise überdeckt.
50. Verfahren nach einem der Aspekte 44 bis 50, ferner mit dem Schritt räumlich gleichmäßiges Benetzen der mindestens einen Probe.
51. Verfahren nach einem der Aspekte 44 bis 51, ferner mit dem Schritt Erzeugung des Aerosols (16) über der mindestens einen Probe.
52. Verfahren nach einem der Aspekte 44 bis 52, ferner mit dem Schritt Bildung von Wirbeln in der Aerosolwolke, vorzugsweise durch Auftreffen der Wolke auf die Probe und/oder auf eine die Probe bereitstellende Positionierungsvorrichtung (8).
53. Verfahren nach einem der Aspekte 44 bis 53, ferner mit dem Schritt Bildung eines Nebels und gravitationsbedingtes Absinken des Nebels auf die mindestens eine Probe.
54. Verfahren nach einem der Aspekte 51 bis 54, wobei der Schritt gleichmäßiges Benetzen bedeutet, dass bei Verwendung einer Positionierungsvorrichtung für mehr als eine Probe, , die maximal 5 cm² groß ist, die Proben mit der annähernd gleichen Masse an Substanz belegt werden, wobei die Variabilität der Massen, insbesondere das 95% Confidence Level, um den Mittelwert zwischen den Proben weniger als 40%, bevorzugt weniger als 30%, besonders bevorzugt weniger 20% beträgt und ganz besonders bevorzugt weniger als 10% beträgt.
55. Verfahren nach einem der Aspekte 51 bis 55, ferner mit dem Schritt Bereitstellen einer Probe auf einer Positionierungsvorrichtung, vorzugsweise an der LFG.
56. Verfahren nach einem der Aspekte 44 bis 56 und weiter mit dem Schritt Bereitstellen einer Aerosolisierungsvorrichtung (14), vorzugsweise luftstrom-freien Vernebelungseinrichtung zur Erzeugung des Aerosols (16).
57. Verfahren nach einem der Aspekte 44 bis 57, wobei das Aerosol eine pharmazeutisch wirksame Substanz oder eine toxische Substanz aufweist, beispielsweise wasserlösliche anorganische und/oder organische Substanzen, Peptide und/oder Proteine.
58. Verfahren nach einem der Aspekte 44 bis 58, wobei das Aerosol natürlich vorkommende oder absichtlich erzeugte Nanopartikel und/oder Schwebeteilchen aus anorganischen und/oder organischen Substanzen, beispielsweise Rußpartikel, Metall- und Metalloxidpartikel, Kohlenstoffnanoröhrchen, Liposomen, und/oder Gelatinepartikel, aufweist.
59. Verfahren nach einem der Aspekte 44 bis 59, wobei eine gleichmäßige Benetzung der Probe in weniger als 12 min, bevorzugt weniger als 6 min, besonders bevorzugt weniger als 3 min erreicht wird.
60. Verwendung einer Vorrichtung nach einem der Aspekte 1 bis 42 zur Durchführung eines Verfahrens nach einem der Aspekte 44 bis 59.

Die Verfahrensschritte erfolgen vorzugsweise, aber nicht notwendigerweise in der beschriebenen Reihenfolge.

Die vorliegende Erfindung, das heißt die vorliegende erfindungsgemäße Vorrichtung und das erfindungsgemäße Verfahren, ermöglicht eine schnelle, effiziente und gleichmäßige Benetzung bzw. Belegung von Proben, vorzugsweise Zellen an der Luftflüssigkeitsgrenzschicht (LFG) mit einem Aerosol, insbesondere mit Flüssigaerosolen, weiterhin insbesondere mit Flüssigaerosolen mit pharmazeutische Substanzen. Durch den beschriebenen Transport der Substanzen in einem Aerosol mittels bspw. Wolkenkonvektion, insbesondere ohne Erzeugung oder Nutzung einer externen Luftströmung in die oder aus der Vorrichtung oder ohne inertiale Impaktion bzw. Ansprayen, können Nachteile des Standes der Technik vermieden und Vorteile realisiert werden. So kann bei der vorliegenden Erfindung ohne großen technischen Aufwand eine relative Luftfeuchtigkeit nahe 100% nahezu konstant erreicht werden, was beispielsweise zu guten Kultivierungsbedingungen für Zellen, einer stark verlangsamten oder völlig verhinderten Verdunstung der Tröpfchen und einer großen Tröpfchendepositionseffizienz (zwischen 70 und 95% auf dem Bodenbereich) führen kann. Dies kann insbesondere erreicht werden, in dem der Wassergehalt in der Wolke so hoch ist, dass schon die Verdunstung von prozentual kleinen Flüssigkeitsmengen (<5%) zu saturierten Bedingungen führt. Dies ermöglicht es insbesondere, bei der vorliegenden Erfindung auf ein aufwendiges, teures und fehleranfälliges Feuchtigkeitskontrollsystem zu verzichten. Ein weiterer Vorteil der vorliegenden Erfindung besteht darin, dass keine aktive Homogenisierung des Aerosols notwendig ist. Bei bekannten Systemen muss ein derartiges Aerosol beispielsweise aktiv durch Vermischen beispielsweise durch einen Ventilator, erzielt werden. Dies ist hier nicht notwendig, da eine homogene und gleichmäßige Verteilung unter Nutzung von wolkenphysikalischen Effekten erreicht werden kann.

Weiterhin führt beispielsweise die schnelle Depositionsdauervon vorzugsweise weniger als zwölf Minuten und bis hin zu weniger als drei Minuten dazu, dass die Probe nicht lange in der Expositionskammer verweilen muss. Dies kann insbesondere bei biologischen Systemen, beispielsweise Zellen, besonders vorteilhaft sein, da diese sehr empfindlich auf Veränderungen in ihrer Umgebung reagieren. Diese hohe Empfindlichkeit ist, insbesondere bei längeren Expositionszeiten, die Begründung für die Verwendung einer Thermostatisierung des Systems. Die vorliegende Erfindung übertrifft die bekannten Systeme insbesondere sowohl hinsichtlich Effizienz, Dosisgenauigkeit und Gleichverteilung der Substanzen bei gleichzeitig deutlich verbesserter Handhabbarkeit.

Weiter ist es bevorzugt, dass die Vorrichtung steril betrieben werden kann. Beispielsweise die leicht zugängliche Bauweise, und die geringe Anzahl von Baukomponenten sowie die Verwendung von alkoholpersistenten Materialien erlauben einfaches Sterilisieren aller aerosol- und zellexponierten Oberflächen. Darüber hinaus ist die Vorrichtung kompakt genug, dass sie in handelsübliche Sterilbanken passt. Dies ist ein Vorteil gegenüber den meisten bekannten Systemen.

Insbesondere bzw. zusammenfassend lässt sich feststellen, besonders im Vergleich zu bisherigen Systemen, dass die vorliegende Erfindung vorteilhafter Weise ein luftstrom-freies System, also insbesondere ohne Luftzu- und Abflüsse, mit sehr kurzen Expositionszeiten (von weniger als 3-12 min anstelle von mehreren Tagen, oder Stunden und mindestens 30 min im Stand der Technik) bei vergleichsweise hohem Substanztransport (ca. 0,5µl Substanz pro cm² Probe pro Minute) zu den Proben ermöglicht, was einen hohen Probendurchsatz erlaubt. Nur eine geringe, wenn überhaupt, Intervention bspw. bzgl. CO₂-Versorgung, der Temperierung für ein Handling der Zellen außerhalb des Brutschranks etc. ist erforderlich. Auch werden ungewöhnlich hohe Depositionseffizienzen erreicht (über 70%, vorzugsweise ca. 75-95% und weiter bevorzugt nahezu 100% des Aerosols erreichen den Bodenbereich, der die Probenvorrichtung mit den Proben enthält), die bei wiederholter Applikation ein hohes Maß an Reproduzierbarkeit aufweisen mit einer Abweichung von 25%, bevorzugt, 20% und besonders bevorzugt 10% von der mittleren Dosis (95% Confidence Lebel), bei einer gleichzeitig hohen Gleichverteilung des deponierten Aerosols auf der/n Probe/n. Die Variabilität der Dosis um den Mittelwert (95% Confidence Level) in 6 Transwell Inserts einer 6-Well Mikrotiterplatte beträgt weniger als 40%, bevorzugt weniger als 30% und besonders bevorzugt weniger als 20%. Auch ist ein vollständig steriles Arbeiten für eine Weiterkultivierung oder wiederholte Expositionen möglich. Das System kann zudem modular konzipiert werden und bei Bedarf einfach um weitere Komponenten ergänzt werden, bspw. um eine Heizvorrichtung, (Quarzkristall)Mikrowaage, Laserstrahl oder dergleichen. Eine besonders gute Anwendbarkeit für biologische Proben wird insbesondere durch die geringe Expositionszeit und den geringen Stress durch die sanfte Art der Aerosoldeposition (langsame "Wolkendeposition") erreicht.

### Kurzbeschreibung der Figuren

Die Erfindung wird nun anhand beispielhafter Ausführungsformen in Bezug auf die beigefügten Zeichnungen beschrieben. Es zeigen:
- Figur 1: eine erste Ausführungsform einer Vorrichtung zur Benetzung mindestens einer Probe mit Aerosol,
- Figur 2: eine weitere Ausführungsform einer Vorrichtung zur Benetzung einer Positioniervorrichtung mit bis zu sechs Proben mit Aerosol,
- Figuren 3a-3c: Schritte eines Verfahrens mit einer Vorrichtung zur Benetzung einer Positioniervorrichtung mit bis zu sechs Proben mit Aerosol, und
- Figur 4: Dosis-abhängige anti-inflammatorische Wirkung des Proteasominhibitors Bortezomib auf eine humane, nicht-polarisierte, pulmonale Zelllinie (A549) an der LFG, die mit Tumornekrosefaktor α (TNFα) inflammatorisch stimuliert wurden.

### Ausführliche Beschreibung der Figuren

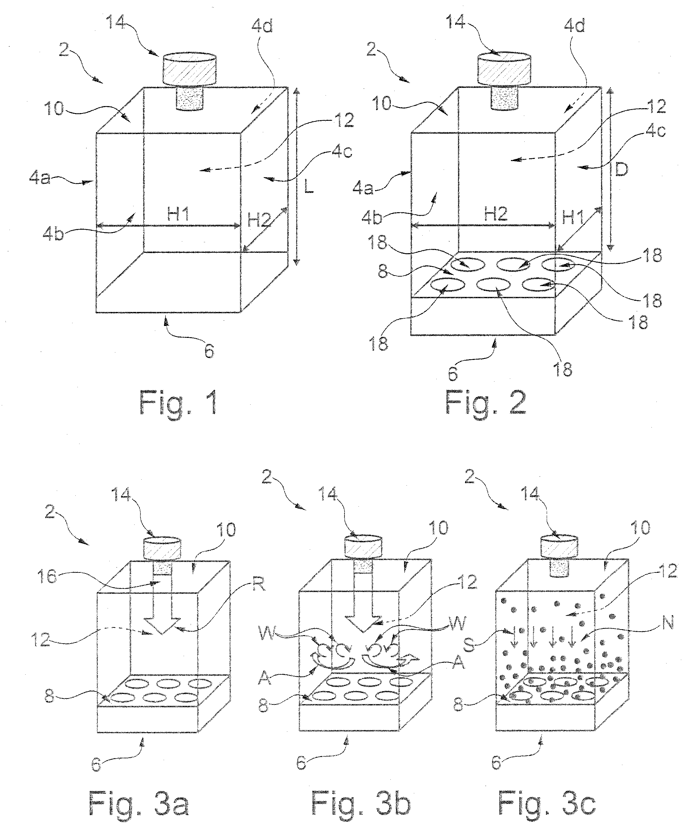

Nachfolgend werden eine bevorzugte Vorrichtung und ein bevorzugtes Verfahren beispielhaft beschrieben. Figur 1 zeigt eine erste Ausführungsform einer Vorrichtung 2 zur Benetzung mindestens einer Probe mit einer Substanz. Die Vorrichtung 2 weist im Wesentlichen vertikale Seitenwände 4a, 4b, 4c und 4d auf. In der dargestellten Ausführungsform bilden diese im Wesentlichen vertikalen Seitenwände 4a, 4b, 4c und 4d im Wesentlichen rechte Winkel zueinander. Obwohl dies bevorzugt ist, ist dies nicht notwendig. Alternativ ist es möglich, dass Vorrichtung 2 eine andere Anzahl an im Wesentlichen vertikalen Seitenwänden 4a, 4b, 4c und 4d aufweist. So ist zum Beispiel denkbar, dass lediglich drei Seitenwände vorgesehen sind, die jeweils einen Winkel von etwa 60° zueinander bilden. Ebenso können mehr als vier Seitenwände vorgesehen sein, zum Beispiel 5, 6 usw.. Ebenso ist es denkbar, dass nur eine durchgehende Wand, beispielsweise in Form eines Zylinders vorgesehen ist. Auch können die Winkel zwischen den vertikalen Seitenwänden 4a, 4b, 4c und 4d unterschiedlich sein. Schließlich kann es bevorzugt sein, dass die Seitenwände nicht im Wesentlichen Vertikal sind und beispielsweise einen sich, vorzugsweise nach oben verjüngenden, Pyramidenstumpf oder Kegelstumpf bilden.

Weiterhin weist die Vorrichtung 2 einen im Wesentlichen horizontalen Bodenbereich bzw. Unterbau 6 auf, der Platz für die Positionierung mindestens einer Probe bietet. Dieser ist vorzugsweise durch die Seitenwände 4a, 4b, 4c und 4d bzw. deren untere Enden begrenzt. Der Bodenbereich bzw. Unterbau 6 stellt eine funktionalisierbare Bodenfläche dar. In einer Ausführungsform, wie in Figur 1 gezeigt, weist dieser Bodenbereich, abgesehen von den unteren Bereichen der Seitenwände 4a, 4b, 4c und 4d keinen Stoff bzw. keine Materie auf - d. h. der gesamte Bodenbereich 6 kann als ausgesparter Bereich realisiert sein bzw. ist als durch die Seitenwände definierte und insbesondere seitlich begrenzte Öffnung ausgebildet. Alternativ kann der Bodenbereich eine Bodenplatte oder weitere Elemente, nicht dargestellt, umfassen.

Die Vorrichtung 2 umfasst ferner einen Deckelbereich 10. Dieser Deckelbereich 10 liegt dem Bodenbereich hier vertikal gegenüber. Auch der Deckelbereich 10 ist vorzugsweise durch die Seitenwände 4a, 4b, 4c und 4d begrenzt. Ebenso wie der Bodenbereich 6 kann auch der Deckelbereich 10, abgesehen von den oberen Bereichen der Seitenwände 4a, 4b, 4c und 4d keine Materie bzw. keinen Stoff aufweisen. In anderen Worten kann der Deckelbereich 10 also als ein ausgesparter Bereich ausgebildet sein bzw. ist als durch die Seitenwände definierte und insbesondere seitlich begrenzte Öffnung ausgebildet. Es ist bevorzugt, dass der Deckelbereich 10 nicht komplett als ausgesparter Bereich ausgebildet ist. So kann der Deckelbereich 10 auch Material, beispielsweise einen Deckel aufweisen. In so einem Fall ist der Deckelbereich 10 jedoch mit einer Aussparung bzw. einer Öffnung versehen, die vorzugsweise in der Mitte von Deckelbereich 10 angeordnet ist, um die Verneblungseinheit anzuschließen. Diese Öffnung bzw. diese Aussparung kann weiterhin verschließbar ausgestaltet sein.

Durch die im Wesentlichen vertikalen Seitenwände 4a, 4b, 4c und 4d sowie durch Bodenbereich 6 und Deckelbereich 10 wird ein räumlicher Bereich abgetrennt, der eine Expositionskammer 12 bildet. Dieser kann eine im Wesentlichen quadratische oder rechteckige Grundfläche haben. Andere Formen können bevorzugt sein. Weiterhin weist die dargestellte Ausführungsform eine Aerosolisierungsvorrichtung und hier eine Verneblungsvorrichtung 14 zur Erzeugung eines Substanz-Gasgemisches auf. Diese Aerosolisierungsvorrichtung 14 ist in der Nähe des Deckelbereichs 10 angeordnet. In anderen Worten hat die Aerosolisierungsvorrichtung 14 in etwa den gleichen Abstand zum Bodenbereich 6 wie Deckelbereich 10.

Die Vernebelungseinrichtung 14 ist angepasst, Aerosol in Richtung des Bodenbereichs 6 freizusetzen, wie nachfolgend weitergehend beschrieben. Weiter ist die Aerosolisierungsvorrichtung 14 über dem Bodenbereich 6 angeordnet und zwar derart, dass die Aerosolisierungsvorrichtung 14 den Bodenbereich 6 in einer vertikalen Draufsicht zumindest teilweise überdeckt und vorzugsweise mittig dazu angeordnet ist.

Der Bodenbereich 6 ist zur Aufnahme einer plattenartigen Positionierungsvorrichtung 8 zur Positionierung der Probe(n) angepasst. Dies kann z. B. durch eine geeignete Ausgestaltung und Dimensionierung des Bodenbereichs 6 realisiert werden - in Figur 2 ist eine bevorzugte Ausführungsform gezeigt, die auch die plattenartige Positionierungsvorrichtung 8 für bis zu sechs Proben zeigt, die in Ausnehmungen bzw. Aushebungen 18 angeordnet sind. Diese Aushebungen 18 können die Proben und/oder Halterungen für die Proben aufnehmen. Hierbei kann die Vorrichtung bzw. deren Bodenbereich 6 über die Positionierungsvorrichtung gestülpt werden oder auf die Positionierungsvorrichtung aufgesetzt werden. Alternativ weist der Bodenbereich einen, vorzugsweise abnehmbaren, Boden auf, auf dem die Positionierungsvorrichtung angeordnet werden kann oder der als Positionierungsvorrichtung ausgestaltet ist.

Das Aerosol weist feste und/oder flüssige, vorzugsweise flüssige, Aerosolpartikel auf. Ganz besonders bevorzugt ist es weiterhin, dass das Aerosol eine pharmazeutische oder eine toxische Substanz aufweist.

Typischerweise ist die Aerosolisierungsvorrichtung insbesondere ihr Auslass in die Expositionskammer vertikal um eine Distanz D von der Positionierungsvorrichtung und insbesondere von den Proben entfernt (Figur 2). Diese Distanz D kann beispielsweise etwa zwischen 5 cm und 50 cm, bevorzugt etwa zwischen 7 cm und 30 cm, besonders bevorzugt etwa zwischen 9 cm und 20 betragen.

Darauf abgestimmt weisen die vertikalen Seitenwände 4a, 4b, 4c und 4d typischerweise eine vertikale Ausdehnung L (Figur 1) etwa zwischen 8 cm und 53 cm, besonders bevorzugt etwa zwischen 10 cm und 33 cm, und insbesondere zwischen 12 cm und 23 cm auf.

Die horizontale Ausdehnung H1 und H2 der Seitenwände 4a, 4b, 4c und 4d und damit auch die Seitenlänge des Bodenbereichs 6 zur Positionierung der Positionierungsvorrichtung 8 können derart ausgestaltet sein, dass eine erste horizontale Seitenlänge H1 im Bereich zwischen 5 cm und 100 cm, bevorzugt zwischen 7 cm und 50 cm, und besonders bevorzugt zwischen 8 cm und 25 cm beträgt und eine zweite horizontale Seitenlänge H2 zwischen 4 cm und 80 cm, bevorzugt zwischen 5 cm und 40 cm, und besonders bevorzugt zwischen 6 cm und 20 cm beträgt.

Typischerweise ist die Dimensionierung derart ausgestaltet, dass der Bodenbereich 6 zur Aufnahme einer, zwei, drei oder vier Mikrotiterplatten ausgebildet ist. So kann die Positionierungsvorrichtung 8, wie in Figur 2 gezeigt, insbesondere eine, zwei, drei oder vier Mikrotiterplatten aufweisen und insbesondere aus einer, zwei, drei oder vier Mikrotiterplatten bestehen. Besonders bevorzugt ist die Vorrichtung 2 und insbesondere der Abstand der im Wesentlichen vertikalen Seitenwände 4a, 4b, 4c und 4d derart ausgestaltet, dass genau eine ganze Anzahl solcher Standard-Mikrotiterplatten bündig innerhalb der Seitenwände 4a, 4b, 4c und 4d angeordnet werden kann.

Weiter kann die Vorrichtung 2 und insbesondere die Dimensionierung und die zu verwendenden Positionierungsvorrichtungen 8 derart ausgestaltet sein, dass sie zur Benetzung einer Vielzahl von Proben, vorzugsweise wie bereits beschrieben, geeignet sind. In Figur 2 ist eine solche Ausgestaltung gezeigt, in der die Vorrichtung zur Benetzung von maximal 6 Proben ausgebildet ist.

Besonders bevorzugt ist es, dass die Probe Zellen und vorzugsweise Epithelzellen aufweist - daher ist die Vorrichtung 2 besonders bevorzugt geeignet, diese Zellen bzw. Epithelzellen zu benetzen. Weiterhin ist es besonders bevorzugt, dass die Positionierungsvorrichtung 8 derart angepasst ist, dass die Proben, z. B. die Zellen und vorzugsweise die Epithelzellen eine Luftflüssigkeitsgrenzschicht (LFG) bilden. Vorzugsweise weist eine Positioniervorrichtung Vertiefungen auf, in denen Zellproben mittels Einsätzen, bspw. Transwell-Inserts, für die LFG Kultivierung angeordnet werden können.

Darstellungsgemäß ist die Aerosolisierungsvorrichtung 14 in der Nähe des Deckelbereichs 10 angeordnet. In anderen Worten hat die Aerosolisierungsvorrichtung 14 also in etwa einen gleichen Abstand zum Bodenabschnitt 6 bzw. zur Positionierungsvorrichtung 8 wie der Deckelbereich 10.

Hierbei kann die Aerosolisierungsvorrichtung 14 beispielsweise knapp oberhalb des Deckelbereichs 10 oder auch knapp unterhalb des Deckelbereichs 10 angeordnet sein - beispielsweise mit einem Abstand zwischen 0 cm und 10 cm, bevorzugt zwischen 3 cm und 7 cm und insbesondere ca. 5 cm darüber oder darunter.

Es ist besonders bevorzugt, dass die Vorrichtung 2 genau eine Aerosolisierungsvorrichtung 14 aufweist. Allerdings kann die Erfindung auch ebenso mit mehr als einer Aerosolisierungsvorrichtung 14, z. B. mit 2, 3, 4 oder 5 Aerosolisierungsvorrichtungen 14 realisiert werden (nicht dargestellt).

Es ist besonders bevorzugt, dass die Aerosolisierungsvorrichtung 14 einen Schwingmembranvernebler aufweist und vorzugsweise ein solcher ist. Typischerweise weist die Aerosolisierungsvorrichtung 14 eine perforierte (Schwing)Membran auf, die zumindest im Wesentlichen parallel zur Horizontalen ausgerichtet ist. Typischerweise ist die Aerosolisierungsvorrichtung 14 angepasst, Aerosolpartikel mit einer mittleren Größe zwischen 1 µm und 15 µm, bevorzugt zwischen 2 µm und 10 µm, besonders bevorzugt zwischen 3 µm und 7 µm zu erzeugen bzw. freizusetzen.

Weiterhin ist es bevorzugt, dass die Aerosolisierungsvorrichtung 14, insbesondere in Draufsicht, in etwa mittig über dem Bodenbereich 6 bzw. der Positionierungsvorrichtung 8 angeordnet ist.

Insbesondere erlaubt es die Positionierung der Aerosolisierungsvorrichtung 14 im Bereich bzw. in der Nähe des Deckelbereichs 10, dass das Aerosol 16 ohne einen zusätzlichen Luftstrom freigesetzt und in die Expositionskammer eingeleitet werden kann. In anderen Worten erlaubt es die genaue Anordnung der Aerosolisierungsvorrichtung 14, nämlich derart, dass die Aerosolisierungsvorrichtung 14 den Bodenbereich 6 bzw. die plattenartige Positionierungsvorrichtung 8 in einer vertikalen Draufsicht zumindest teilweise überdeckt, dass das Aerosol 16 die Aerosolisierungsvorrichtung 14 mit einer Geschwindigkeit verlassen kann, die groß genug ist, die Wolke rasch und zusammenhängend in die Nähe des Bodenbereichs zu transportieren, aber nicht so hoch, dass die Aerosole direkt bei der ersten Annäherung an den Bodenbereich dort impaktieren. Insbesondere können die freigesetzten Flüssigkeitspartikel die Aerosolisierungsvorrichtung 14 mit einer mittleren Geschwindigkeit von zwischen ca. 5 cm bis 1000 cm/s, bevorzugt ca. 10 bis 500 cm/s, besonders bevorzugt zwischen 30 und 130 cm/s verlassen. Insbesondere ist es bevorzugt, dass die freigesetzten Aerosolpartikel nach dem Verlassen der Aerosolisierungsvorrichtung 14 nur noch der Wirkung der Massenträgheit und der Schwerkraft unterliegen, d. h. dass keine weiteren externen Kräfte auf die Wolke einwirken. Bevorzugt bilden die Aerosolpartikel nach dem Verlassen eine Aerosolwolke, wie eingangs beschrieben. Ein zusätzlicher Luftstrom ist bevorzugt nicht notwendig.

Die Vorrichtung besteht bevorzugt aus 3 Teilen (Fig. 1 bzw. 2). Nämlich einer (Standard-)Mikrotiterplatte als Positionierungsvorrichtung, in der die Proben (Zellen) kultiviert werden; einem Schwingmembranvernebler zur Erzeugung einer Aerosol- vorzugsweise Tröpfchenwolke; und einer Expositionskammer, wie vorstehend beschrieben.

Vorzugsweise ist die quaderförmige Expositionskammer im Grundriss so angelegt, dass genau eine Standard-Mikrotiterplatte hineinpasst (12,8 cm x 8,6 cm). Die Kammer ist etwa 16,0 cm hoch und an ihrer Decke ist zentral ein kommerziell verfügbarer Schwingmenbranvemebler angebracht (Höhe über der Kammerdecke hier vorzugsweise ca. 5cm), der mittels einer schwingenden perforierten Membran eine Flüssigkeit direkt in eine dichte Aerosol- bzw. Tröpfchenwolke verwandelt. Der Vernebler ist nach unten gerichtet, sodass die Wolke direkt über der Standard-Mikrotiterplatte gebildet wird. Die Kammer besteht aus durchsichtigem Polycarbonat (Makrolon), das leicht mit Alkohol sterilisiert werden kann. Das gesamte System ist sehr kompakt mit einem Gesamtgewicht von ca. 0.8 kg.

Mit Verweis auf die Figuren 3a-3c wird im Folgenden auch ein Verfahren zur Benetzung mindestens einer Probe mit einem Aerosol, insbesondere mittels einer Vorrichtung 2 wie vorgehend beschrieben, beispielhaft dargestellt.

Das Verfahren, insbesondere der Zellexposition von Epithelzellen in der LFG, wie dargestellt lässt sich in drei Phasen gliedern, nämlich (1) Erzeugung einer Aerosolwolke, vorzugsweise durch Vernebelung einer Flüssigkeit und "freier Fall" der erzeugten Wolke; (2) Nebelbildung und Homogenisierung (Gleichverteilung) der Wolke in der Expositionskammer durch Vortexbildung; und (3) Absinken des Aerosolnebels auf die Zellen. Fig. 3a entspricht dabei Phase (1), Fig. 3b Phase (2) und Fig. 3c ist Phase (3) zuzuordnen.

Gemäß Phase 1 wird zunächst eine, beispielsweise pharmazeutische oder toxische Substanz der Aerosolisierungsvorrichtung 14 zugeführt, beispielsweise durch einpipettieren (typischerweise etwa 200µl). Die Aerosolisierungsvorrichtung wird aktiviert und generiert eine Aerosol-, vorzugsweise eine Tröpfchenwolke. Die Wolke kann eine Anfangsgeschwindigkeit haben und ist insbesondere so dicht, dass die Gesetze der Wolkenbewegung gelten, was insbesondere zu einem raschen "freien" Fall der Wolke (Gravitation) führt und damit einem schnellen konvektiven Transport der Substanz bzw. des Wirkstoffs zu den Zellen.

In Phase 2, am Boden angekommen, wird die Wolke gleichmäßig in alle Richtungen seitlich abgelenkt A, wodurch symmetrische Vortices W gebildet werden, die zu einer horizontal gleichmäßigen Verteilung der Wolke und damit der Bildung eines (Tröpfchen-)Nebels N im unteren Teil der Kammer führt. Bei weiterer Vernebelungsaktivität füllt der Nebel die Kammer allmählich von unten nach oben, wobei der Nebel wegen der Gravitation immer in Bodennähe am dichtesten ist.

Schließlich, in der 3. Phase, nach Beendigung der Vernebelung sinkt der Nebel, wie durch Pfeile S in Figur 3c angedeutet, aufgrund der Gravitation in ca. 3 min und weniger auf die am Boden liegende Probe(n), die vorzugsweise in einer Standard-Mikrotiterplatte als darin kultivierte LFG-Zellen vorliegt/vorliegen, die dadurch gleichmäßig benetzt wird/werden. Bei der Bildung eines solchen Nebels N ist allgemein die Partikelkonzentration nahe des Bodenbereichs 6 bzw. nahe der Positionierungsvorrichtung 8 höher als weiter davon entfernt - dies ist durch die zunehmend dunkle Schraffierung in Figur 3c angedeutet.

Dadurch lässt sich insbesondere eine rasche, hoch effiziente und horizontal gleichmäßige Belegung von Zellen an der Luft Flüssigkeitsgrenzschicht (LFG) erreichen. Die Handhabung der Vorrichtung verlangt keinerlei Expertenwissen über Aerosol-oder Wolkenphysik, ist einfach zu bedienen und zu reinigen sowie wenig fehler- und störanfällig.

Weiterhin können auch weitere Elemente, wie vorstehend, insbesondere im Zusammenhang mit Figur 1 und 2, beschrieben, selbstverständlich in dem nun beschriebenen Verfahren benutzt werden. Ferner wird das Verfahren vorzugsweise mit der beschriebenen Vorrichtung 2 durchgeführt.

Allgemein tragen vorzugsweise die Größe und Dichte der Wolke, sowie die exakten Dimensionierungen der vertikalen Seitenwände 4a, 4b, 4c und 4d, des Bodenbereichs 6 bzw. der Positionierungsvorrichtung 8, sowie der Abstand D der Aerosolisierungsvorrichtung 14 von der Positionierungsvorrichtung 8, die vertikale Ausdehnung L der Seitenwände 4a, 4b, 4c und 4d Parameter, dazu bei, ein schnelles und gleichmäßiges Benetzen der Probe mit der Substanz zu gewährleisten bzw. zu verbessern.

Die erfindungsgemäße Vorrichtung bzw. das erfindungsgemäße Verfahren erlauben insbesondere die einfache und verlässliche Erzeugung eines gleichmäßigen vorzugsweise ca. 15 - 100µm dünnen Flüssigkeitsfilms auf ebenen (und strukturierten) Oberflächen. Damit ist sie potentiell nicht nur für LFG-Zellkulturen anwendbar, sondern generell auch für die Erzeugung von mikrometerdünnen gleichmäßigen Schichten auf horizontalen Oberflächen.

Vorteilhaft ist hierbei insbesondere die eigenaktive bzw. inhärente Homogenisierung der Benetzungen, die beispielsweise ohne zusätzliche Mittel, bspw. erzeugen eines elektrischen Feldes oder eines zusätzlichen Luftstroms oder Mischungssystems, auskommen kann. Mit dem Verfahren wird insbesondere eine gleichmäßige Verteilung der Aerosolpartikel auf den Zellen erreicht. Dies wird insbesondere durch die Nutzung von wolkenphysikalischen Effekten erreicht, die durch ein optimales Design hinsichtlich der folgenden Parameter begünstigt werden: (1) Geometrische Dimension der Expositionskammer; (2) Positionierung des Verneblers (3) Substanz- bzw. Flüssigkeitsabgabe des Verneblers pro Zeiteinheit (Output rate) sowie (4) ein geeigneter Durchmesser und eine geeignete Anfangsgeschwindigkeit der vom Vernebier generierten Aerosolwolke.

### Ergebnisse von exemplarischen Tests

Durchgeführte Experimente mit der Vorrichtung 2, insbesondere mit einer solchen wie oben beschrieben, haben folgende Kenngrößen ergeben:
Bei Verwendung der Vorrichtung 2 wird ein hoher Depositionsfaktor von mehr als 0,8 bzw. mehr als 80% erzielt. In anderen Worten werden mehr als 80% einer eingesetzten Flüssigkeit bzw. eines Aerosols auf die Bodenplatte (ggf. mit Positionierungsvorrichtung 8) deponiert. Dies wurde festgestellt mit der Vernebelung einer Fluoresceinlösung als Surrogat für einen pharmazeutischen Wirkstoff. Dies wurde aus Daten für mindestens 6 Proben mit einer Fläche von je ca. 4.5cm² geschlossen, die an mindestens 6 verschiedenen Stellen über die Bodenplatte verteilt wurden, und auf die Gesamtfläche der Bodenplatte (125 cm²) umgerechnet wurden.

Weiterhin ist die applizierte Gesamtdosis sehr reproduzierbar. Bei wiederholter Vernebelung von 200 µl Fluoresceinlösung wurde eine Abweichungen von maximal ca. +/- 10% (95% Confidence Level) von der im Mittel auf die 6 Proben deponierten Gesamtdosis gemessen.

Weiterhin wurde eine gleichmäßige Verteilung des Wirkstoffs über die verschiedenen Wells (mit 6-well Transwell Insert) einer Mikrotiterplatte gemessen. Die Abweichung der Dosis in einzelnen Wells von der mittleren Dosis in allen Wells war insbesondere kleiner als +/- 20% (95% confidence level).

Weiterhin wurden Wirkstoffdepositionsraten von bis zu 0,5µl/cm²/min gemessen - hierbei handelt es sich um das Volumen des Wirkstoffs (Substanz) pro zellbedeckter Fläche pro Zeit. Weiterhin konnten bei Verwendung der erfindungsgemäßen Vorrichtung und dem erfindungsgemäßen Verfahren keine negativen Effekte auf biologische Proben aufgrund des Handlings während der Exposition festgestellt werden. Insbesondere zeigen Zellen einer humanen pulmonalen Zelllinie (A549), die bei Verwendung der erfindungsgemäßen Vorrichtung mit einer (nicht-toxischen) physiologischen Kochsalzlösung beaufschlagt wurden, keine Reduktion der Lebensfähigkeit (WST-1) oder ein Einsetzen von Nekrose (LDH). Dies ist ein Vorteil für den Einsatz des Verfahrens mit biologischen Proben.

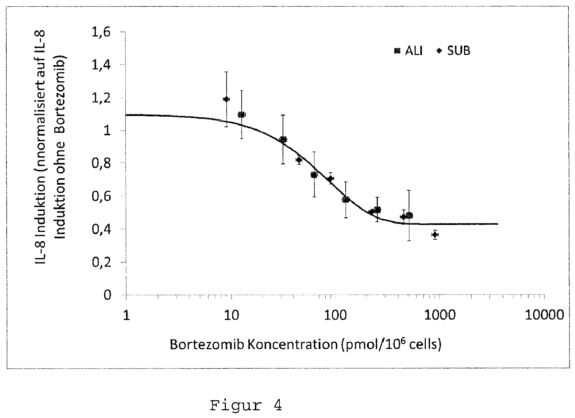

Darüber hinaus wurde gezeigt, dass A549 Zellen in Vorrichtung 2 nicht nur lebensfähig sind, sondern auch für die systematische Untersuchung der Wirksamkeit von neuen Wirkstoffen verwendet werden können (Drug Screening). Verwendet man eine nicht-polarisierte Lungenzelllinie (hier: A549), also Zellen, die sich aber auf der Luftseite nicht anders organisieren als auf der Flüssigkeitsseite, muss man erwarten, dass die biologische Antwort der Zellen an der LFG (in Vorrichtung 2) genau so ist, wie unter submersen Kulturbedingungen, bei denen der Wirkstoff in das Medium einpipettiert wird. Verwendet man dagegen polarisierte Lungenepithelzellen (z.B. Primärzellen), ist mit deutlichen Unterschieden in der biologischen Antwort zu rechnen. Nach Stimulation von A549 Zellen mit dem Tumornekrosefaktor α (TNFα) ist das Interleukin 8 (IL-8) als inflammatorischer Marker relativ zum Basalwert (nichtstimulierte Zellen) ca. 7-fach induziert (entspricht dem Wert 1 in Figur 4). Die inflammatorische Wirkung kann durch Applikation eines anti-inflammatorischen Wirkstoffs (hier Bortezomib; ein Proteasominhibitor) dosisabhängig um bis zu einem Faktor 2 reduziert werden. Dabei zeigt sich, dass die Dosis-Wirkungskurve nach Verabreichung des Wirkstoffs in aerosolisierter Form mit Vorrichtung 2, identisch zur Referenzkurve ist, die durch Pipettieren des Wirkstoffs direkt auf die Zellen erstellt wurde. Dies zeigt exemplarisch, dass mit Vorrichtung 2, aerosolisierte Wirkstoffe dosisgenau und ohne Verlust an Bioaktivität (z.B. durch Scherspannungen bei der Vernebelung) auf humane pulmonale Zellen an der LFG aufgebracht werden können. Damit ist Vorrichtung 2 für das systematische Screening von Substanzen hinsichtlich ihres Potentials als Wirkstoff in aerosoliserter Form geeignet.

Figur 4 zeigt die Dosis-abhängige anti-inflammatorische Wirkung des Proteasominhibitors Bortezomib auf eine humane, nicht-polarisierte, pulmonale Zelllinie (A549) an der LFG, die mit Tumornekrosefaktor α (TNFα) inflammatorisch stimuliert wurden. Die mit Vorrichtung 2 erstellte Dosis-Wirkungskurve (rot, ALI) unterscheidet sich dabei nicht von der Referenzkurve (blau, SUB), die durch Pipettieren des Wirkstoffs auf die submers kultivierten Zellen gemessen wurde. Damit ist Vorrichtung 2 als Werkzeug für quantitatives Wirkstoffscreening für aerosolisierte, flüssige Wirkstoffe validiert.

Die Erfindung umfasst ebenfalls die genauen oder exakten Ausdrücke, Merkmale, numerischen Werte oder Bereiche usw., wenn vorstehend oder nachfolgend diese Ausdrücke, Merkmale, numerischen Werte oder Bereiche im Zusammenhang mit Ausdrücken wie z.B. "etwa, ca., um, im Wesentlichen, im Allgemeinen, zumindest, mindestens" usw. genannt wurden (also "etwa 3" soll ebenfalls "3" oder "im Wesentlichen radial" soll auch "radial" umfassen - und umgekehrt). Der Ausdruck "bzw." bedeutet überdies "und/oder".

## Patentansprüche

1. Vorrichtung (2) zur Benetzung mindestens einer Probe mittels eines Aerosols, wobei die Vorrichtung (2) aufweist
Seitenwände (4a, 4b, 4c, 4d),
einen Bodenbereich (6), der durch die Seitenwände (4a, 4b, 4c, 4d) begrenzt ist, wobei der Bodenbereich (6) zur Aufnahme von oder zum Zusammenwirken mit einer Positionierungsvorrichtung (8) zur Positionierung der Probe angepasst ist,
einem Deckelbereich (10), der dem Bodenbereich (6) vertikal gegenüberliegt, und der durch die Seitenwände (4a, 4b, 4c, 4d) begrenzt ist
wobei die Vorrichtung (2) eine Expositionskammer (12) bildet,
eine Aerosolisierungsvorrichtung (14) zur Erzeugung eines Aerosols (16), die in der Nähe des Deckelbereichs (10) angeordnet ist,
wobei die Aerosolisierungsvorrichtung (14) über dem Bodenbereich (6) angeordnet ist, derart dass die Aerosolisierungsvorrichtung (14) den Bodenbereich (6) in einer vertikalen Draufsicht zumindest teilweise überdeckt
wobei die Vorrichtung (2) und/oder die Aerosolisierungsvorrichtung (14) ausgebildet sind, eine Wolke zu bilden, und
wobei die Wolke Aerosolpartikelvolumen von mindestens 10⁻⁴% aufweist,
wobei die Vorrichtung (2) **dadurch gekennzeichnet ist, dass** die Vorrichtung eingerichtet ist, ohne externen Luftstrom in oder aus der Vorrichtung (2), betrieben zu werden.

2. Vorrichtung (2) nach Anspruch 1, wobei die Aerosolisierungsvorrichtung mindestens einen Vernebler aufweist.

3. Vorrichtung nach einem der vorhergehenden Ansprüche, wobei die Aerosolisierungsvorrichtung (14) so orientiert ist, dass die initiale Bewegungsrichtung der Wolke mit der Vertikalen einen Winkel kleiner als 30° einschließt und bevorzugt im Wesentlichen parallel zur Vertikalen ist.

4. Vorrichtung nach einem der vorhergehenden Ansprüche, wobei in einer vertikalen Draufsicht die Aerosolisierungsvorrichtung in etwa mittig über dem Bodenbereich bzw. der Positionierungsvorrichtung angeordnet ist.

5. Vorrichtung nach einem der vorhergehenden Ansprüche, wobei die Vorrichtung derart ausgebildet ist, dass eine von der Aerosolisierungsvorrichtung initial erzeugte Wolke so beschaffen ist, dass die mittlere Fallgeschwindigkeit der am Anfang der Exposition erzeugten Wolke größer als1cm/s, bevorzugt größer als 3 cm/s, besonders bevorzugt größer als10 cm/s ist.

6. Vorrichtung nach einem der vorhergehenden Ansprüche, wobei die Vorrichtung derart ausgebildet ist, dass die von der Aerosolisierungsvorrichtung erzeugte Wolke so beschaffen ist, dass die Wolke unbehindert fallen kann und somit ein rascher Transport des Aerosols zum Bodenbereich gewährleistet wird.

7. Vorrichtung nach einem der vorhergehenden Ansprüche, wobei die Expositionskammer derart dimensioniert ist, dass die horizontale Querschnittsfläche der Expositionskammer in der oberen Hälfte der Kammer mehr als 2-fach, bevorzugt 2,5-fach, besonders bevorzugt mehr als 3-fach so groß wie die Querschnittsfläche der initial erzeugten Wolke ist.

8. Vorrichtung (2) nach einem der vorhergehenden Ansprüche, mit einer Positionierungsvorrichtung (8) zur Positionierung einer Probe.

9. Vorrichtung nach einem der vorhergehenden Ansprüche, wobei die Vorrichtung und/oder die Positionierungsvorrichtung ausgebildet ist/sind, eine Probe aufzunehmen bzw. zu benetzen.

10. Vorrichtung nach einem der vorhergehenden Ansprüche, wobei die Probe biologisches Material und/oder Werkstoffproben aufweist.

11. Vorrichtung (2) nach einem der vorhergehenden Ansprüche, wobei die Positionierungsvorrichtung Elemente zur Messung und/oder Regulierung einer Temperatur, beispielsweise eine elektrische Heizung oder eine mit Hilfe eines Wasserkreislaufes betriebene Temperiervorrichtung, Elemente zur Versorgung der Probe, bspw. mit Nährmedium, und/oder Elemente zum Messen des Gewichts auf einer oder mehreren Proben deponierten Substanz, beispielsweise eine oder mehrere Quartzkristall-Mikrowaagen, aufweist.

12. Verfahren zur Benetzung mindestens einer Probe mittels eines Aerosols mittels einer Vorrichtung nach einem der vorhergehenden Ansprüche, wobei das Verfahren die Schritte aufweist:
Bereitstellen mindestens einer Probe,
Erzeugen einer Wolke (16) oberhalb der mindestens einen Probe an einer Position, die den Bodenbereich (6) in einer vertikalen Draufsicht zumindest teilweise überdeckt und
Absinkenlassen der Wolke in Richtung (R) der Probe
wobei die Wolke ein Aerosolpartikelvolumen von mindestens 10⁻⁴% aufweist.

13. Verfahren nach Anspruch 12, ferner mit dem Schritt Ausformung von Vortices, die die Wolke in einen Nebel überführen, der die Expositionskammer in horizontaler Richtung (H₁-H₂) gleichförmig ausfüllt.

14. Verfahren nach einem der Ansprüche12 bis 13, ferner mit dem Schritt gravitationsbedingtes Absinken des Nebels.

15. Verfahren nach einem der Ansprüche12 bis 14, ferner mit dem Schritt Bildung von Wirbeln in der Aerosolwolke, vorzugsweise durch Auftreffen der Wolke auf den Bodenbereich und/oder auf eine die Probe bereitstellende Positionierungsvorrichtung (8).

16. Verfahren nach einem der Ansprüche12 bis 15, ferner mit dem Schritt Bildung eines Nebels und gravitationsbedingtes Absinken des Nebels auf mindestens eine Probe.

17. Verfahren nach einem der Ansprüche12 bis16 und weiter mit dem Schritt Bereitstellen einer Aerosolisierungsvorrichtung (14), vorzugsweise einer luftstrom-freien Vernebelungseinrichtung zur Erzeugung des Aerosols (16).

18. Verfahren nach einem der Ansprüche12 bis 17, ferner mit dem Schritt räumlich gleichmäßiges Benetzen der mindestens einen Probe.

19. Verwendung einer Vorrichtung nach einem der Ansprüche1 bis 11 zur Durchführung eines Verfahrens nach einem der Ansprüche12 bis 18.

## Claims

1. Device (2) for wetting at least one sample by means of an aerosol, wherein the device (2) comprises:
side walls (4a, 4b, 4c, 4d),
a bottom portion (6) bounded by the side walls (4a, 4b, 4c, 4d), the bottom portion (6) being adapted to receive or cooperate with a positioning device (8) for positioning the sample,
a cover portion (10) arranged vertically opposite to the bottom portion (6) and bounded by the side walls (4a, 4b, 4c, 4d),
the device (2) forming an exposure chamber (12),
an aerosolization device (14) for producing an aerosol (16) which is arranged in the vicinity of the cover portion (10),
wherein the aerosolization device (14) is arranged above the bottom portion (6) such that the aerosolization device (14) at least partially covers the bottom portion (6) in a vertical plan view,
wherein the device (2) and/or the aerosolization device (14) are configured to form a cloud, and
wherein the cloud has aerosol particle volumes of at least 10⁻⁴%,
the device (2) being **characterized in that** the device is arranged to operate without external air flow into or out of the device (2).

2. Device according to claim 1, wherein the aerosolization device comprises at least one nebulizer.

3. Device according to one of the preceding claims, wherein the aerosolization device (14) is oriented so that the initial direction of movement of the cloud with forms an angle of less than 30° with the vertical and preferably is substantially parallel to the vertical.

4. Device according to one of the preceding claims, wherein in a vertical plan view, the aerosolization device is arranged approximately centrally above the bottom portion or the positioning device.

5. Device according to one of the preceding claims, wherein the device is configured such that a cloud initially generated by the aerosolization device is such that the average fall velocity of the cloud generated at the beginning of the exposure is greater than 1 cm/s, preferably greater than 3 cm/s, more preferably greater than 10 cm/s.

6. Device according to one of the preceding claims, wherein the device is configured such that the cloud generated by the aerosolization device is such that the cloud can fall in an unobstructed manner and thus a rapid transport of the aerosol to the bottom portion is ensured.

7. Device according to one of the preceding claims, wherein the exposure chamber is dimensioned such that the horizontal cross-sectional area of the exposure chamber in the upper half of the chamber is more than 2 times, preferably 2.5 times, more preferably more than 3 times as large as the cross-sectional area of the initially generated cloud.

8. Device according to one of the preceding claims, with a positioning device (8) for positioning a sample.

9. Device according to one of the preceding claims, wherein the device and/or the positioning device is/are adapted to receive or to wet a sample.

10. Device according to one of the preceding claims, wherein the sample comprises biological material and/or material samples.

11. Device according to one of the preceding claims, wherein the positioning device comprises elements for measuring and/or regulating a temperature, for example an electric heater or a temperature control operated by means of a water circulation, elements for sustaining the sample, e.g. with nutrient medium, and/or elements for measuring the weight of substance deposited on one or more samples, for example one or more quartz crystal microbalances.

12. A method for wetting at least one sample by means of an aerosol by means of a device according to one of the preceding claims, the method comprising the steps:
providing at least one sample,
generating a cloud (16) above the at least one sample at a position which at least partially covers the bottom region (6) in a vertical plan view, and
dropping the cloud in direction (R) of the sample, wherein the cloud has an aerosol particle volume of at least 10⁻⁴%.

13. The method of claim 12, further comprising the step of forming vortices that convert the cloud into a mist that uniformly fills the exposure chamber in the horizontal direction (H₁-H₂).

14. The method according to any one of claims 12 to 13, further comprising the step of gravitational descent of the mist.

15. The method of any one of claims 12 to 14, further comprising the step of forming vortices in the aerosol cloud, preferably by impinging the cloud on the bottom portion and/or on a sample positioning device (8).

16. A method according to any one of claims 12 to 15, further comprising the step of forming a mist and gravitationally lowering the mist onto at least one sample.

17. A method according to any one of claims 12 to 16 and further comprising the step of providing an aerosolization device (14), preferably an airflow-free nebulizer for generating the aerosol (16).

18. The method of claim 12, further comprising the step of spatially uniformly wetting the at least one sample.

19. Use of a device according to one of claims 1 to 11 for carrying out a method according to one of claims 12 to 18.

## Revendications

1. Dispositif (2) de mouillage d'au moins une sonde au moyen d'un aérosol, le dispositif (2) présentant
des parois latérales (4a, 4b, 4c, 4d),
une zone de fond (6), qui est délimitée par les parois latérales (4a, 4b, 4c, 4d), la zone de fond (6) étant adaptée pour recevoir un dispositif de positionnement (8) destiné à positionner la sonde ou pour coopérer avec ce dernier,
une zone de couvercle (10) qui est en regard verticalement de la zone de fond (6) et qui est délimitée par les parois latérales (4a, 4b, 4c, 4d),
le dispositif (2) formant une chambre d'exposition (12), un dispositif d'aérosolisation (14) pour produire un aérosol (16), qui est disposé à proximité de la zone de couvercle (10),
le dispositif d'aérosolisation (14) étant disposé au-dessus de la zone de fond (6) de telle manière que le dispositif d'aérosolisation (14) recouvre au moins à certains endroits la zone de fond (6) en vue de dessus verticale,
le dispositif (2) et/ou le dispositif d'aérosolisation (14) sont conçus pour former un nuage, et
le nuage présentant un volume de particules d'aérosol d'au moins 10⁻⁴ %, le dispositif (2) étant **caractérisé en ce que** le dispositif est conçu pour fonctionner sans flux d'air externe dans le dispositif (2) ou à partir de ce dernier.

2. Dispositif (2) selon la revendication 1, le dispositif d'aérosolisation présentant au moins un nébulisateur.

3. Dispositif selon l'une quelconque des revendications précédentes, le dispositif d'aérosolisation (14) est orienté de telle manière que la direction du mouvement initiale du nuage forme avec la verticale un angle inférieur à 30° et de préférence sensiblement parallèle à la verticale.

4. Dispositif selon l'une quelconque des revendications précédentes, dans une vue du dessus verticale, le dispositif d'aérosolisation étant disposé à peu près au milieu au-dessus de la zone de fond ou du dispositif de positionnement.

5. Dispositif selon l'une quelconque des revendications précédentes, le dispositif étant conçu de telle manière qu'un nuage produit initialement par le dispositif d'aérosolisation soit obtenu de sorte que la vitesse moyenne de chute du nuage produit au début de l'exposition soit supérieure à 1cm/s, de préférence supérieure à 3 cm/s, de préférence encore supérieure à 10 cm/s.

6. Dispositif selon l'une quelconque des revendications précédentes, le dispositif étant conçu de telle manière que le nuage produit par le dispositif d'aérosolisation soit obtenu de sorte que le nuage puisse chuter sans entrave et que l'aérosol puisse être ainsi transporté rapidement vers la zone de fond.

7. Dispositif selon l'une quelconque des revendications précédentes, la chambre d'exposition étant dimensionnée de telle manière que la surface de section transversale horizontale de la chambre d'exposition dans la moitié supérieure de la chambre est 2 fois, de préférence 2,5 fois, de préférence encore plus de 3 fois plus grande que la surface de section transversale du nuage produit initialement.

8. Dispositif (2) selon l'une quelconque des revendications précédentes, comprenant un dispositif de positionnement (8) pour positionner une sonde.

9. Dispositif selon l'une quelconque des revendications précédentes, le dispositif et/ou le dispositif de positionnement est/sont conçu(s) pour recevoir ou mouiller une sonde.

10. Dispositif selon l'une quelconque des revendications précédentes, la sonde présente un matériau biologique et/ou des sondes de matière.

11. Dispositif (2) selon l'une quelconque des revendications précédentes, le dispositif de positionnement présentant des éléments pour mesurer et/ou réguler une température, par exemple un chauffage électrique ou un dispositif de thermorégulation fonctionnant par circulation d'eau, des éléments destinés à alimenter en sonde ou en milieu de culture, et/ou des éléments pour mesurer le poids de substance déposée sur une ou plusieurs sondes par exemple une ou plusieurs microbalance à quartz.

12. Procédé de mouillage d'au moins une sonde au moyen d'un aérosol au moyen d'un dispositif selon l'une quelconque des revendications précédentes, le procédé comprenant les étapes consistant à :
préparer au moins une sonde,
produire un nuage (16) au-dessus de l'au moins une sonde dans une position qui recouvre au moins à certains endroits la zone de fond (6) dans une vue de dessus verticale et
laisser s'affaisser le nuage dans la direction (R) de la sonde,
le nuage présentant un volume de particule d'aérosol d'au moins 10⁻⁴ %.

13. Procédé selon la revendication 12, comprenant en outre l'étape de formation de vortex qui transfèrent le nuage dans un brouillard qui remplit uniformément la chambre d'exposition dans la direction horizontale (H₁-H₂).

14. Procédé selon l'une quelconque des revendications 12 à 13, comprenant en outre l'étape consistant en l'affaissement gravitationnel du brouillard.

15. Procédé selon l'une quelconque des revendications 12 à 14, comprenant en outre l'étape consistant en la formation de tourbillons dans le nuage d'aérosol, de préférence par la rencontre du nuage sur la zone de fond et/ou sur le dispositif de positionnement (8) préparant la sonde.

16. Procédé selon l'une quelconque des revendications 12 à 15, comprenant en outre l'étape consistant la formation d'un nuage et l'affaissement gravitationnel du brouillard sur au moins une sonde.

17. Procédé selon l'une quelconque des revendications 12 à 16 et comprenant en outre l'étape consistant en la préparation d'un dispositif d'aérosolisation (14), de préférence un

18. Procédé selon l'une quelconque des revendications 12 à 17, comprenant en outre l'étape consistant à mouiller uniformément dans l'espace l'au moins une sonde.

19. Utilisation d'un dispositif selon l'une des revendications 1 à 11 destinée à exécuter un procédé selon l'une des revendications 12 à 18.
